# EUROPEAN PATENT APPLICATION

(11) **EP 3 348 278 A1**
(43) Date of publication of application: **18.07.2018**
(21) Application number: 16844231.7
(22) Date of filing: 30.08.2016
(51) Int. Cl.: A61K 45/00, A61K 31/7088, A61K 31/7105, A61K 48/00, A61P 35/00, A61P 43/00, C12N 15/113, C12Q 1/68, G01N 33/15, G01N 33/50

(54) **EXOSOME SECRETION INHIBITOR**

(30) Priority: 09.09.2015 JP 2015177552
(71) Applicant: Theoria Science Inc., Tokyo 101-0032 (JP)
(72) Inventor: OCHIYA, Takahiro, Tokyo 104-0053 (JP); YOSHIOKA, Yusuke, Tokyo 120-0021 (JP); KOSAKA, Nobuyoshi, Tokyo 195-0053 (JP)
(74) Representative: Vossius & Partner Patentanwälte Rechtsanwälte mbB
(86) International application number: PCT/JP2016/075324
(87) International publication number: WO 2017/043370

(57) **Abstract**

An exosome secretion inhibitor containing a substance suppressing expression of NAPG, HINT3 or GXYLT1 gene, or a substance suppressing activity of NAPG, HINT3 or GXYLT1 protein. It is possible to suppress secretion of exosome, thereby making it possible to suppress proliferation or metastasis of cancer cells, so that the substance suppressing expression of the gene or activity of the protein can be used as an active ingredient of a pharmaceutical composition suitably used in treatment of cancer or suppression of cancer metastasis, and also the substance can provide a carcinostatic agent having a new action mechanism which specifically suppresses expression of the gene or activity of the protein.

SEQUENCE LISTING

<110>  SHIONOGI&Co.,Ltd.
       THEORIA Science Inc.

<120>  An inhibitor for exosome secretion

<130>  16-043-PCTJP

<150>  JP 2015-177552
<151>  2015-09-09

<160>  6

<170>  PatentIn version Ver. 3.5.1

<210> 1
<211> 939
<212> RNA
<213> Homo sapiens

<300>
<308> Genbank/CR536554

<400> 1
atggcggctc agaagataaa cgaggggctg gaacacctcg ccaaagcaga gaaatacctg        60

aaaactggtt ttttaaaatg gaagccagat tatgacagtg ccgcttctga atatggaaaa       120

gcagctgttg cttttaaaaa tgccaaacag tttgagcaag caaaagatgc ctgcctgagg       180

gaagctgttg cccatgaaaa taatagggct ctttttcatg ctgccaaagc ttatgagcaa       240

gctggaatga tgttgaagga gatgcagaaa ctaccagagg ccgttcagct aattgagaag       300

gccagcatga tgtatctaga aaacggcacc ccagacacag cagccatggc tttggagcga       360

gctggaaagc ttatagaaaa tgttgatcca gagaaggctg tacagttata tcaacagaca       420

gctaatgtgt ttgaaaatga agaacgctta cgacaggcag ttgaattact aggaaaagcc       480

tccagactac tagtacgagg acgtaggttt gatgaggcgg cactctctat tcagaaagaa       540

aaaaatattt ataaggaaat tgagaattat ccaacttgtt ataagaaaac aattgctcaa       600

gtcttagttc atctacacag aaatgactat gtagctgcag aaagatgtgt ccgggagagc       660

tatagcatcc ctgggttcaa tggcagtgaa gactgtgctg ccctggaaca gcttcttgaa       720

ggttatgacc agcaagacca agatcaggtg tcagatgtct gcaactcacc gcttttcaag       780

tacatggaca atgattatgc taagctgggc ctgagtttgg tggttccagg agggggaatc        840

aagaagaaat cacctgcaac accacaggcc aagcctgatg gtgtcactgc cacggctgct       900

gatgaagagg aagatgaata ctcaggagga ctatgctag                              939

<210> 2
<211> 312
<212> PRT
<213> Homo sapiens

<300>
<308> Genbank/CAG38791

<400> 2
Met Ala Ala Gln Lys Ile Asn Glu Gly Leu Glu His Leu Ala Lys Ala
1               5                   10                  15

Glu Lys Tyr Leu Lys Thr Gly Phe Leu Lys Trp Lys Pro Asp Tyr Asp
            20                  25                  30

Ser Ala Ala Ser Glu Tyr Gly Lys Ala Ala Val Ala Phe Lys Asn Ala
        35                  40                  45

Lys Gln Phe Glu Gln Ala Lys Asp Ala Cys Leu Arg Glu Ala Val Ala
    50                  55                  60

His Glu Asn Asn Arg Ala Leu Phe His Ala Ala Lys Ala Tyr Glu Gln
65                  70                  75                  80

Ala Gly Met Met Leu Lys Glu Met Gln Lys Leu Pro Glu Ala Val Gln
                85                  90                  95

Leu Ile Glu Lys Ala Ser Met Met Tyr Leu Glu Asn Gly Thr Pro Asp
            100                 105                 110

Thr Ala Ala Met Ala Leu Glu Arg Ala Gly Lys Leu Ile Glu Asn Val
        115                 120                 125

Asp Pro Glu Lys Ala Val Gln Leu Tyr Gln Gln Thr Ala Asn Val Phe
    130                 135                 140

Glu Asn Glu Glu Arg Leu Arg Gln Ala Val Glu Leu Leu Gly Lys Ala
145                 150                 155                 160

Ser Arg Leu Leu Val Arg Gly Arg Arg Phe Asp Glu Ala Ala Leu Ser
                165                 170                 175

Ile Gln Lys Glu Lys Asn Ile Tyr Lys Glu Ile Glu Asn Tyr Pro Thr
            180                 185                 190

Cys Tyr Lys Lys Thr Ile Ala Gln Val Leu Val His Leu His Arg Asn
        195                 200                 205

Asp Tyr Val Ala Ala Glu Arg Cys Val Arg Glu Ser Tyr Ser Ile Pro
    210                 215                 220

Gly Phe Asn Gly Ser Glu Asp Cys Ala Ala Leu Glu Gln Leu Leu Glu
225                 230                 235                 240

Gly Tyr Asp Gln Gln Asp Gln Asp Gln Val Ser Asp Val Cys Asn Ser
                245                 250                 255

Pro Leu Phe Lys Tyr Met Asp Asn Asp Tyr Ala Lys Leu Gly Leu Ser
            260                 265                 270

Leu Val Val Pro Gly Gly Gly Ile Lys Lys Lys Ser Pro Ala Thr Pro
        275                 280                 285

Gln Ala Lys Pro Asp Gly Val Thr Ala Thr Ala Ala Asp Glu Glu Glu
    290                 295                 300

Asp Glu Tyr Ser Gly Gly Leu Cys
305                 310

<210> 3
<211> 3396
<212> RNA
<213> Homo sapiens

<300>
<308> Genbank/NM_138571

<400> 3
cattaccaac gaggcgcagg ggtcaggacg actctcggca gcgccattgc gcgccctcta 60

gtggcagccg gttttgaggc cggcctccgg ctttgaagtt cctcaccgcg tctccttccc 120

tctccccaaa gcctggatca ccgcccagcg tcaggcgagg ggcgacgtct cgaggtaaaa 180

cggaggaggt gcgggacgcg gagactgcgc gggcccggta gccctggaga ggccgaggct 240

ctaggccgcg aggggcgggt gcaatggcgg aggaacaggt gaaccgcagc gccggcctgg 300

cccccgactg tgaggcctcg gcgactgcag aaactacggt ttcctcagtg gggacctgtg 360

aagccgctgg caagtcacca gagcccaagg actacgacag cacctgcgtg ttctgccgga 420

tcgcggggcg gcaggacccg ggcaccgaac tcctgcactg cgagaatgag gacctaattt 480

gcttcaaaga tatcaaacca gcagcaactc atcattatct tgtggtgcca aagaagcata 540

ttggaaactg cagaactcta aggaaagatc aagtagaact ggttgagaac atggtaactg 600

ttggaaaaac cattcttgaa agaaataatt tcactgactt cacgaatgtg aggatgggtt 660

ttcatatgcc accattctgt tccatttccc acttgcacct tcatgttctg gcaccagtgg 720

atcagcttgg cttcttatcc aagttggttt atagagtcaa ttcctattgg tttatcacag 780

ctgatcactt gattgaaaaa ctaagaacat gaaaatgtca agagtggaag atttttctaa 840

tcttggttca gcatgaagtg gtatttaggt cccttttaag tctaattgca attttaagat 900

ttgttgggtt ttatgagagg ctgttactta gtggccttaa atcttttctg aatgtctgtt 960

tcctaagatc tgtgatacag ttatgtgaat attttgttac tgacttgttt caatggttac 1020

ttgtataagg attttatata tatgatacta tagataaaat cctatttaag acaaattctg 1080

ttaatcaaca agggctctgt atttttttaa gttaaaatat tttcatttct cagtaagtag 1140

tcagttataa tagtgattta tttatgaaga ataaactact atagaaagta ctttgtggga 1200

acctttatta ctgtgaagga gaaaaatagc tatttcttga cagttctata gttatacaca 1260

taaattttca aaataaagaa taccatacta cacaatgatc tctgattttg ctgagggaag 1320

gaattatata tgtgccactt tttcctcaaa tttcagatgc cagttttcac tttttgaagc 1380

attgcatctt tttaataaat ctatcctgaa agctatataa tatatataat ttatatcaat 1440

gaatttacaa taactatcaa attggctatc agaattcact gtaatggaat tttgcctcta 1500

ataacataaa gggggaagaa acataaagtg gttctcttaa taaaactagc agaaaatgtt 1560

gctttatggc atgtttttgt atcaaagaga aatcagaggt taaactttgc ttttatcatg 1620

aaaagggcct gatttgaagg tagagatagg cttagatgag aaaaattgat aacgcaatgt 1680

taatactatt gatatttcat aggtatattt tgttagaaat tggttttttg gtgctaatct 1740

ataggcacaa tagtgaatat gaatgaagag taggtgctgc aagttaatcc tacattgtgc 1800

cagtgtatat tagtaccatg taaatgaata tgaataaatg aacatgagga ttaacgaact 1860

gttgaaaata ggctgagata ctagttttct ttctttttta gagggggcat gatattaaag 1920

atagttttaa gtaacaaatt acttattaca aagttacaac atgtggaatc aatagtttta 1980

tttgttcatt aatttattca ttcagcaaac atttgttgaa catttaatgt gtaccagata 2040

ttgccaggat caggaaataa agaactaaaa atggacattc agactgcaat tgctagtaca 2100

ggggtcaaac aaaatcagtg attagaattt agtgcgttaa gttccctgat gccttcacag 2160

ccagaagagg ctgtgaaggg ataaacactt ctgagagtgg gtggtagtag aactgagtat 2220

tcaagactga atgttaggca ggtagacagt gactggttag gctgagaaac ttacaagtat 2280

tttcgttgag ttctgcttcc actattattt actttacaat ggatatgaag ttcagatttc 2340

atcttattta ctgaaggtgg agaaaggatg tggaagtagg ggttatgggc tctcaaaagt 2400

agatttagag agattttttt atcactgttt tatgatatag ttcactgagc acttacatag 2460

attaacagtt acaagtttcc ataaatcagt tagaatatga ctagcttcag ggaaggaatt 2520

ttcaacaact gcaatctttg attgttttac tgtgggaact tgcagtgata taattgacaa 2580

cattatttaa caataatagg taaagtaggc cgggtgtggt ggctcacgcc tgtaatccta 2640

gcactttggg aggccgaggt gggtggatca caaggtcagg agttcgagac cagcctggcc 2700

aatatggtga aaccccgtct cttctaaaaa aatacaaaaa ttagccgggt atggtggcgc 2760

atgcctgtag tcccagctac tggggaggct gaggcaagag aattgcttga actcgggagg 2820

cggaggtcgc agtgagccga gatcgtgcca ctgcactcca gcctgggtga cagagtgaga 2880

ctttgtctca aagaaaaaaa aacaataata ggtaaagtat ttaaggaatt ctcagatgcc 2940

ttatggacct cttcaaaaat gtggttagcc aattgattca acttttatag gtttatatgc 3000

tctcatgtaa acctatttgt ttccgagatc agacgagatc gggcgcgttc agggtggtat 3060

ggccgtagac aacctatttg ttaaaaaccc ttggaaacac atcttttagt agaggttctt 3120

atttacttat aaagattaat aatcaaatta ctacaattaa tatccattta gccttttcag 3180

tagtctaaat atttggtaac tccctagcca tctataacac tagttcaaaa caaggctaat 3240

tcataacctg tcttaaaaat gtttgaagtg gtatcaagag cattgtttat gcatgttgtg 3300

atactatttg tggaactgtt taatgagcta tttcaaactg ctagaagaaa atgaaataaa 3360

aatggaaaaa tactgtcaaa aaaaaaaaaa aaaaaa 3396

<210> 4
<211> 182
<212> PRT
<213> Homo sapiens

<300>
<308> Genbank/NP_612638

<400> 4
Met Ala Glu Glu Gln Val Asn Arg Ser Ala Gly Leu Ala Pro Asp Cys
1               5                   10                  15

Glu Ala Ser Ala Thr Ala Glu Thr Thr Val Ser Ser Val Gly Thr Cys
            20                  25                  30

Glu Ala Ala Gly Lys Ser Pro Glu Pro Lys Asp Tyr Asp Ser Thr Cys
        35                  40                  45

Val Phe Cys Arg Ile Ala Gly Arg Gln Asp Pro Gly Thr Glu Leu Leu
    50                  55                  60

His Cys Glu Asn Glu Asp Leu Ile Cys Phe Lys Asp Ile Lys Pro Ala
65                  70                  75                  80

Ala Thr His His Tyr Leu Val Val Pro Lys Lys His Ile Gly Asn Cys
                85                  90                  95

Arg Thr Leu Arg Lys Asp Gln Val Glu Leu Val Glu Asn Met Val Thr
            100                 105                 110

Val Gly Lys Thr Ile Leu Glu Arg Asn Asn Phe Thr Asp Phe Thr Asn
        115                 120                 125

Val Arg Met Gly Phe His Met Pro Pro Phe Cys Ser Ile Ser His Leu
    130                 135                 140

His Leu His Val Leu Ala Pro Val Asp Gln Leu Gly Phe Leu Ser Lys
145                 150                 155                 160

Leu Val Tyr Arg Val Asn Ser Tyr Trp Phe Ile Thr Ala Asp His Leu
                165                 170                 175

Ile Glu Lys Leu Arg Thr
            180

<210> 5
<211> 7509
<212> RNA
<213> Homo sapiens

<300>
<308> Genbank/NM_173601

<400> 5
ggcggtgtcc aggcttccgg gcgggtagtc cttcggctcc ggagccgcga ctgcgctcgc      60

ctaggtggtg ggcggggagg gaaggaaggg agcgggcgca aggcggcggc ggcggctgcg     120

ggtgaggaac ttgttgcgcc cgcggctgcg cggtgcccct ccctccgctc cagttcgtcg     180

gggcgggcgc ggcggcggcg gcgaaggagg agcgcggccg gggcgatgcg gcgctacctg     240

cgcgtcgtgg tgctgtgtgt ggcctgcggc ttctgctcgc tcctttacgc tttcagccag     300

ctcgccgtgt ccctggaaga aggaacgggc ggcggtggcg ggaagccgca ggccgcggtg 360

gcttcctggc tcgcgggcgg cggacgcggc gccgtgagag gcgccggcgt cgcgggcccc 420

gcagcgcatc ccggcgtgtc ggacaggtgt aaagatttct ctctgtgtta ctggaatccc 480

tattggatgc tgccctctga tgtttgtgga atgaactgct tttgggaagc ggcttttagg 540

tacagtctga aaatacagcc tgttgagaaa atgcatctag ctgtagttgc ctgtggtgaa 600

agactggaag aaactatgac catgttgaag tcagctatca ttttcagcat caaacctctt 660

caattccata tttttgctga agatcagcta catcatagct ttaaaggcag acttgacaac 720

tggtcatttc tacaaacatt taattatacg ttatacccca taacctttcc aagtgagaat 780

gcagcagagt ggaaaaaact ctttaaacca tgtgcttcgc agagattgtt cttgccgtta 840

atcctgaaag aagttgactc actattgtat gtcgacactg atatcctttt tttacgacca 900

gttgatgata tttggtcttt actaaagaaa tttaattcca cacaaattgc tgcaatggca 960

ccagaacatg aggaacctcg aataggatgg tataatcgct ttgctaggca tccatattat 1020

ggaaaaactg gagtaaactc tggagttatg ttgatgaaca tgactcgaat gagaaggaag 1080

tatttcaaga atgatatgac aactgtacga ctacaatggg gagatatact tatgccattg 1140

cttaaaaaat acaaactaaa catcacatgg ggtgatcaag atctattgaa tatcgtgttt 1200

tttcataatc cagaaagcct tttgtttttt ccgtgtcaat ggaattatcg accagatcat 1260

tgtatatatg gaagcaattg ccaagaagca gaagaaggag gaatctttat tcttcatggg 1320

aacagaggtg tttaccatga cgataagcaa ccagcattta gagctgttta tgaagcactg 1380

agaaattgtt cttttgaaga tgacaacatc cgttccttat taaaaccttt agaactggaa 1440

ctacaaaaaa cagtgcatac atactgtgga aaaatttaca aaatatttat caaacaacta 1500

gcaaaaagtg taagagatcg ttatgccaga tcaccaaagg aaaagtgatt cttggtgact 1560

gcttaatcaa atggatgaaa acaaagaatc agaagataag tgtgaaggaa tcgtcttgga 1620

tgaagtattc aggaaggaat tactcatctc cagaataatt ttttttttcc taaagaagtt 1680

aagtaagcag tattttcagg taatgaagaa taagttaaaa tcttgggcct caacattgaa 1740

cattttttat ctctgatgtt ttgtaatgtt acttgctatc attccagtat tgatgaaaat 1800

actattgaat gggtttaacc tgcagacttc tgttgactca tactctcaag agtggtaggg 1860

gtgtgtagat ggagaaaatg tacctcaaac agtgccaaca ctcaagactg tgagtagagc 1920

aataatttta tgtcagcact aacctcactt taaaagtgtg agaaaaaagt ttgtttacag 1980

gagcagaaac aggtctgttg tttctgaaga aatgtgatgt aactgatgta accattgaca 2040

atctatgtgt gcctttatac atttcatctc tgttttaaaa tatttttatg acaatcatgt 2100

ttaaaattat ttttagatta caagtaagct gcatgttaaa aattgagctg tgtaaggtag 2160

aggaaaaata gtgaaaactt tgggatttta atctgtgtgt gcgtgtttgt gtgtgagaga 2220

gagtgagaaa tgtagtgttt cattttgtat gcattaaact gtcttgccaa aactcagatc 2280

taagattgtt aaatagatat ttggggaatt tttttttaat cactttaaat gaaaaacgtc 2340

agctttactg ggcattctgg aagcaaaata tattatgctg atggtaaagt gagaacctta 2400

agagtatact catgatggtg gaaagtgtga tggaccaaaa tgcataagct atatacttcc 2460

tgtgagtagt aattttagtt acaatggagt agaaaaatat ggtccattta aacttgtctt 2520

ctacctcata acggccttgc aagatacttt tgaagaagca aatgtctaga gccttacttt 2580

aagtaagtta aacaacttaa gtggatgatt tggaataaga ttgttacatc cagctgtaga 2640

aaatgtggtt ttaattgggt tgatgtgcat ttttgaatat cacctaagac agttaatttt 2700

tttaaatctt gagaaattac ttctttggca ttcttgacct gttagctaat ataactatag 2760

attctgaata cttagcatat actatgctgg ggcacatatc ttattttacc aacaatatga 2820

ttatctatat ggttatcttc tttttctccc tgatatttta atgctcaagt agaaaatgga 2880

aaatcatgaa ggaaaaatac aacctgtgaa aattgtggca gttagctttg tgaagacaca 2940

gtgacctctg tggagacatg gaatatgcag aaagagatgg ttagtacagt tgttctgtcg 3000

tctgcagttc agtcagggac aaattgagag aggaaagttt aagcagggtg cattagaagt 3060

tgataaccag tccagcattc ttagaacaga tgggtttaga gaacacgagt tgtagttcct 3120

tggcagaatg ccatggtgaa tataagaaac tgtgaaattg agcttactga gtagaaaatg 3180

agaatactaa aaatgtgaag tttggagggt atgtattaga tcatttctaa tggtcctcaa 3240

ataatgatca ttgcctgcct gactatgtaa gattcttgtt gggagcttaa gaatagattc 3300

ctgagctagg cctttggaga ctctggcatg ctgtgtctag gagggagtct gtaaaacaat 3360

ttttaatgaa gaaatttaaa aattacacaa aactagaata gtgcattggt actcaacatc 3420

cagatctgac acttatcact atcttaccat gtttgcttca tttgtcattt ttttgtttgc 3480

ttaagtatct taaaatccca tacattgtgt tatttcactg ctgtgaacac tttgggtaga 3540

taccacgata tcacacctaa cagaggcagc agcagtcctt cggcttcatc tcatagccag 3600

tccttaagca aatttccctg attatctcaa aaatcatctt ctagttggtt tgttagaatc 3660

aacagaatat aagtcacatg atgatttcgt ttttaacaat cacctcagat gactttgatg 3720

ttagctactt tgagaaccac tggaaattat tttgccaact tgaaagttca agttaggata 3780

ccacaattct tggcacgttt ggtggattat aaatgtgatt ttgaaatatt agaggaagca 3840

tttggggtta atgacaggat atgaatttat attagtaagt ttgataagat aaaatttcct 3900

cagtgaacgg agaatctcag ccccatgggc tctaattgat tgggacttgt gtagagcgtg 3960

gtcacagttt aatccaactg gactgtatgc tgtggtctca ctcatgttgt ggcttattta 4020

cactgtcaca cacactacat tgtggatcag ttggaaggtt cttcatctgg tttatttaga 4080

cctgacctaa ttgatcattt taccataaat tgaattatag tgaaaactgt cttttgggta 4140

ctgtttctac ctaaaattaa aggtatcaca aaatatgtat aataattttt aaatgaattt 4200

tctgaattta tctttagaca aggtaattct tttgaattct aaagaaatgc agttcttgga 4260

gttgatgaga ggttaaatta tagatggaca acttagagaa cgtattccat cttcatataa 4320

atggtctttc ataaaacata gtgtatctat gcagtttata taaactacat gatttattat 4380

ttgagtctat gtattctgac ctcagaacta tctctgggct acagtattac caaagtggga 4440

tgcctttaaa atgcagtcaa acttcatttg tatgaagtag aaaagaactt gggaaacctt 4500

taaaaatata tattgtgatt tatgctttaa gaaaatgcat acttttttct aatgaagagt 4560

agttcataat atataagggt tttaaggccc tgaaataaca ttagtgctta ctcattgctc 4620

tctcatcttg cctccagtca gatgttattg gtatagtgaa aagaagacca gattttgaac 4680

tagatctgga ttctcttttt agtaactcag gcatgttatt tacccttctc tgtgccttag 4740

gtttttaagt acaatcagtg tacccttcaa acctcacaag atgaaactca gaagagataa 4800

tggatttgga agcattttgc gtggcttata ttatgccacg ccagtgttac tgttgaagtt 4860

ctgttttgtt cgttgttttc tgtacagagc gttcagttta ctcaggctga atgttttctg 4920

acatatatct ccagtatgaa tttgtttatg taaggaagag tttttcagga agagaaagta 4980

gagaagaaaa gaatgatgta actaaatcaa aaaagaagga aaagagagga agtgagaaaa 5040

ggggtgagga aagagaaagg agtaagaatg gggatggcaa agggcagagt aagagaaaag 5100

atggaatgag tgtatggaaa attgagaagt agagatggag agaaatggtg tggaaacatt 5160

tactttttga gttgtagccc tcacactaca tcagtttgct tgcctgggct ctctggggtc 5220

aggaaaggtg ctgacttcca tggaatgggg cagcagggcc tgctgtattt ggtttgttag 5280

tactagagtt gtttcccaat caccctactc actgatcatt tagtgccaat tcaacatttt 5340

atgtcttttt tttttagtgg atcccagttt gtgagctagg acctgtggaa atacaagtat 5400

cttccttttt aactttattg ttttagccat cccaagtgtt agactgcaag actaactaca 5460

tattccccag tggaggttgg aagtggaggg gactgcttca ttaggtctga agggggccaa 5520

ggaatgtgtt gcagcagttt tcgcagatga tactgacgag aggccaggtt tgggaatcac 5580

tgccctgcct tggatggcaa attaagcaat aaattacacc tatttattcc tcagttggga 5640

tactttcagt tgaaccattc tgagtagatt taagagaaca aaagacagca gaaatacctt 5700

aaaagatatt attggctgtc ttatttttca gatatttgta tttaataaat accttagtag 5760

tagtcagaac ctctttgaga tgtttttaga aagaccactg atttgttttt taaaataatt 5820

tttttggttc cttagagtag agaagaaaag taggggtgca atatgtcagg tgtggggcca 5880

gttatccatt tattaaatgc aatttcaagt gtcttaaaaa caataattgc cttattgcat 5940

tttaatattt aataaagtag attaaataaa gatttgtatt ataacagtta ttcttttatc 6000

cattgccaaa gcaacccttt tttgcctatt aaaatctggc taaactgttc tgatgttgtt 6060

tcttaattca ttaaacatga aactaggttt tacaaactaa attatataaa gacagaagtc 6120

taaaaatggt accagaaaat ttttttttaa aaaattaatg tgttattcaa atatccagca 6180

attaaagact catgagaaaa acagaatagc atattagatg taacagttta aaatggattg 6240

caaatcaatg tcagacttaa atagaaaata tttattatca cctcatttct accaataatt 6300

tcagatgttt aatgggactt tgaaaagata ggaaaaaata gaaatgaaaa tttatctcac 6360

ctttaatact gtgaatacta tttggagggt ccctggtcag acatttaaat atcaaactta 6420

agaactgaca tggagccttt gattagttta tatacaggat ctgaatattt acacacatag 6480

cacactatag caacctcttg taaaaaatca tgaagataga attcaacagt gtatattcta 6540

ttaaaaagta aattggctgg gtgtggtggc tcatgcatgt aatcccagca ctttgggagg 6600

ccgaggcagg aggatcgctt gagcccaaga gtttgagacc agcctaggtg acaaagtgag 6660

accccatctc tacaaaaaaa ttaaaaaatt atccagatgt ggtggtgttt gcctgcggtc 6720

ctagctacac aggaagttga ggcaggagga ttgcttgagt gcaagagttt gaggctgcag 6780

agagccaaga ttgcaccatt gcactccagc ctgggtgaca gagcgagacc ctgtctcaaa 6840

aaataaagga aattggcttt gactatgaaa aacagccatt ttcaaccttt ttataaaacc 6900

tcttcaggga atattggaat attatttttg tcaggcaaaa ataatccagg gcttgagcat 6960

tgctgtagaa tatgttatct gttcaaaact tagcagagca cgcatgcatc acatgcattg 7020

tcatcagtat ttgagagctg aaccaatggt ttttgatggt tgccgtaaca gaagatctgt 7080

gaagaggatg gtggtttgca cctgttcctg aagtcaaact ggaaaaggtt gcaggtattt 7140

caattttctc aatcattttg cataaatgta ttaatatttt gagtagtaat agttctaaac 7200

ctgcaggaag gcatcattgt atactgtggt atgtagagaa tactactctt acactgctct 7260

ttatcttaaa atgttttggt atactgtgaa tattttcctc taagtatttg gtgtattttg 7320

acagttatta aaggagaata taaaattaaa gattctggtc taaactaaga ataatggaaa 7380

gttgcttcgt cctgccttag aggagttttc agttcatcct cttcttgaga aactatgtgt 7440

tgctgttact aaataaacat ttactcaata aacgttttat ttcctcaaaa aaaaaaaaaa 7500

aaaaaaaaa 7509

<210> 6
<211> 440
<212> PRT
<213> Homo sapiens

<300>
<308> Genbank/NP_775872

<400> 6
Met Arg Arg Tyr Leu Arg Val Val Val Leu Cys Val Ala Cys Gly Phe
1               5                   10                  15

Cys Ser Leu Leu Tyr Ala Phe Ser Gln Leu Ala Val Ser Leu Glu Glu
            20                  25                  30

Gly Thr Gly Gly Gly Gly Gly Lys Pro Gln Ala Ala Val Ala Ser Trp
        35                  40                  45

Leu Ala Gly Gly Gly Arg Gly Ala Val Arg Gly Ala Gly Val Ala Gly
    50                  55                  60

Pro Ala Ala His Pro Gly Val Ser Asp Arg Cys Lys Asp Phe Ser Leu
65                  70                  75                  80

Cys Tyr Trp Asn Pro Tyr Trp Met Leu Pro Ser Asp Val Cys Gly Met
                85                  90                  95

Asn Cys Phe Trp Glu Ala Ala Phe Arg Tyr Ser Leu Lys Ile Gln Pro
            100                 105                 110

Val Glu Lys Met His Leu Ala Val Val Ala Cys Gly Glu Arg Leu Glu
        115                 120                 125

Glu Thr Met Thr Met Leu Lys Ser Ala Ile Ile Phe Ser Ile Lys Pro
    130                 135                 140

Leu Gln Phe His Ile Phe Ala Glu Asp Gln Leu His His Ser Phe Lys
145                 150                 155                 160

Gly Arg Leu Asp Asn Trp Ser Phe Leu Gln Thr Phe Asn Tyr Thr Leu
                165                 170                 175

Tyr Pro Ile Thr Phe Pro Ser Glu Asn Ala Ala Glu Trp Lys Lys Leu
            180                 185                 190

Phe Lys Pro Cys Ala Ser Gln Arg Leu Phe Leu Pro Leu Ile Leu Lys
        195                 200                 205

Glu Val Asp Ser Leu Leu Tyr Val Asp Thr Asp Ile Leu Phe Leu Arg
    210                 215                 220

Pro Val Asp Asp Ile Trp Ser Leu Leu Lys Lys Phe Asn Ser Thr Gln
225                 230                 235                 240

Ile Ala Ala Met Ala Pro Glu His Glu Glu Pro Arg Ile Gly Trp Tyr
                245                 250                 255

Asn Arg Phe Ala Arg His Pro Tyr Tyr Gly Lys Thr Gly Val Asn Ser
            260                 265                 270

Gly Val Met Leu Met Asn Met Thr Arg Met Arg Arg Lys Tyr Phe Lys
        275                 280                 285

Asn Asp Met Thr Thr Val Arg Leu Gln Trp Gly Asp Ile Leu Met Pro
    290                 295                 300

Leu Leu Lys Lys Tyr Lys Leu Asn Ile Thr Trp Gly Asp Gln Asp Leu
305                 310                 315                 320

Leu Asn Ile Val Phe Phe His Asn Pro Glu Ser Leu Phe Val Phe Pro
                325                 330                 335

Cys Gln Trp Asn Tyr Arg Pro Asp His Cys Ile Tyr Gly Ser Asn Cys
            340                 345                 350

Gln Glu Ala Glu Glu Gly Gly Ile Phe Ile Leu His Gly Asn Arg Gly
        355                 360                 365

Val Tyr His Asp Asp Lys Gln Pro Ala Phe Arg Ala Val Tyr Glu Ala
    370                 375                 380

Leu Arg Asn Cys Ser Phe Glu Asp Asp Asn Ile Arg Ser Leu Leu Lys
385                 390                 395                 400

Pro Leu Glu Leu Glu Leu Gln Lys Thr Val His Thr Tyr Cys Gly Lys
                405                 410                 415

Ile Tyr Lys Ile Phe Ile Lys Gln Leu Ala Lys Ser Val Arg Asp Arg
            420                 425                 430

Tyr Ala Arg Ser Pro Lys Glu Lys
        435                 440

## Description

### TECHNICAL FIELD

The present invention relates to an exosome secretion inhibitor. More specifically, the present invention relates to an exosome secretion inhibitor and a pharmaceutical composition, containing a substance suppressing expression of a particular gene or activity of a particular protein, and a method of screening a substance inhibiting exosome secretion, based on fluctuations of expression of a particular gene or activity of a particular protein.

### BACKGROUND ART

Most common features of cancer cells are rapid and uncontrollable proliferation as compared to normal cells. Therefore, much researches have been made on an approach in which a gene to be targeted by cancer therapy is found and proliferation of cancer cells are suppressed by suppressing expression or function of a target gene to treat cancer.

For example, Patent Publication 1 discloses miR-194 as a biomarker associated with the recurrence of liver cancer in a sample isolated from a patient with liver cancer. In addition, Patent Publication 2 describes that autoregulation loop associated with p53/HDM2 in multiple myeloma can be suppressed with miR-194.

In addition, it has been known that expression of NAPG gene is increased in endothelial cells of ovarian cancer (see, Patent Publication 3). It is reported that this NAPG gene is associated with a bipolar disorder or vesiculation in addition to the above disease (see, Non-Patent Publications 1 and 2).

On the other hand, it has been reported that exosome present in body fluid in a living body is secreted from various cells, for example, immunological cells or various cancer cells, and it has been remarked that exosome functions as a mediator of an intercellular communication in a living body and associated with physiological phenomenon and that the exosome is associated with diseases such as cancer.

For example, Non-Patent Publication 3 clarifies that exosome secreted by fibroblasts is involved in bosselation of lung cancer cells. In addition, it has been reported that exosome derived from melanoma promotes metastasis of primary focus, and that exosome which is secreted from metastatic focus by stimulation of neutral sphingomyelinase 2 (nSMase2) further promotes metastasis of cancers (see, Non-Patent Publications 4 and 5).

### PRIOR ART REFERENCES

### PATENT PUBLICATIONS

Patent Publication 1: WO 2012/151212
Patent Publication 2: WO 2012/019053
Patent Publication 3: WO 2008/101118

### NON-PATENT PUBLICATIONS

Non-Patent Publication 1: Neuroscience Letters, 457(2009), 159-162
Non-Patent Publication 2: Genes to Cells, (2005), 10, 989-999
Non-Patent Publication 3: Cell, 151, 1542-1556, December 21, 2012
Non-Patent Publication 4: Nat Med., 2012 June; 18(6):883-891
Non-Patent Publication 5: J. Biol. Chem., 2013, 288:10849-10859

### SUMMARY OF THE INVENTION

### PROBLEMS TO BE SOLVED BY THE INVENTION

While the relationship of each of a biomarker and cancer, and exosome and cancer is clarified, a biomarker regulating secretion of exosome has not yet been known.

An object of the present invention is to provide, as a new approach to cancer therapy, an exosome secretion inhibitor, a pharmaceutical composition, and a method of screening a substance for inhibiting exosome secretion, based on fluctuations of expression of a particular gene or activity of a particular protein by inhibiting secretion of exosome, thereby suppressing proliferation or metastasis of cancer cells to find genes which enable the treatment of cancer.

### MEANS TO SOLVE THE PROBLEMS

The present invention relates to the following [1] to [4]:
[1] An exosome secretion inhibitor containing a substance suppressing expression of NAPG, HINT3 or GXYLT1 gene, or a substance suppressing activity of NAPG, HINT3 or GXYLT1 protein.
[2] A pharmaceutical composition containing a substance suppressing expression of NAPG, HINT3 or GXYLT1 gene, or a substance suppressing activity of NAPG, HINT3 or GXYLT1 protein.
[3] An exosome secretion inhibitor containing miR-194.
[4] A method of screening a substance for inhibiting exosome secretion, including using expression of NAPG, HINT3 or GXYLT1 gene or activity of NAPG, HINT3 or GXYLT1 protein as an index to make a substance suppressing the expression or suppressing the activity a candidate compound.

### EFFECTS OF THE INVENTION

According to the present invention, the exosome secretion can be suppressed by suppressing expression of NAPG, HINT3 or GXYLT1 gene, or suppressing activity of NAPG, HINT3 or GXYLT1 protein, which in turn makes it possible to suppress proliferation or metastasis of cancer cells. Therefore, the substance inhibiting expression of the above gene or activity of the above protein can be used as an active ingredient of a pharmaceutical composition suitably used in the treatment of cancer or suppression of cancer metastasis. The pharmaceutical composition of the present invention provides a carcinostatic agent having a new action mechanism which specifically suppresses expression of a particular gene or activity of the protein. Further, the present invention provides a method of screening an exosome secretion inhibitor which is effective as a substance for the treatment of cancer or a substance for suppression of metastasis using the above gene or protein as a target.

### BRIEF DESCRIPTION OF THE DRAWINGS

[FIG. 1A] FIGs. 1A to D are diagrams showing the results of researching miRNA having an inhibitory action of exosome secretion. (A) is diagrams showing one example of Example 1, which show the results obtained by using siRNA against RAB27A (Applied Biosystems), RAB27B (Applied Biosystems), and nSMase2 (Applied Biosystems) as positive controls. Dotted line in the chart shows the amount of exosome secretion in a group added with negative control siRNA, and broken line shows the amount of exosome secretion in a group added with positive control siRNA against RAB27A.
[FIG. 1B] FIG. 1(B) is graphs showing one example of the results of measurement of the amount of exosome secretion using an anti-CD63 antibody and an anti-CD9 antibody, which show a relative secretion amount to the secretion amount of negative control miRNA. Left graphs are the results obtained by using PC-3ML cells, and right graphs are the results obtained by using MDA-MB-231D3H2LN cells.
[FIG. 1C] FIG. 1(C) is graphs showing one example of the results of comparing the number of exosome secretion particles per cell count. Left graph is the results obtained by using PC-3ML cells, and right graph is the results obtained by using MDA-MB-231D3H2LN cells.
[FIG. 1D] FIG. 1(D) is graphs showing that the number of exosome secretion particles is changed by administration of LNA. Left graph is the results of the exosome secretion amount by carrying out LNA treatment using PC-3ML cells, and right graph shows an expression level of miR-194 itself.
[FIG. 2A] FIGs. 2A to B are diagrams showing the results of identification of target genes of miR-194. (A) shows the results of analyzing and extracting genes which alter depending on the fluctuations of expression of miR-194 using a microarray.
[FIG. 2B] FIG. 2(B) is a graph showing the results of analyzing and comparing an expression level of NAPG which is a candidate of a target gene using real-time PCR method. Left zone is the results obtained by using PC-3ML cells, and right zone is the results obtained by using MDA-MB-231D3H2LN cells.
[FIG. 3A] FIGs. 3A to B are graphs showing the results of evaluations of inhibition of exosome secretion by siRNA. (A) is the results for MDA-MB-231D3H2LN cells, wherein the upper panel shows the results of measurement using an anti-CD9 antibody, and the lower panel shows the results of measurement using an anti-CD63 antibody, respectively. Dotted lines in the graphs show values of negative control (NC1), and broken lines show values of positive control (RAB27A).
[FIG. 3B] FIG. 3(B) is the results for PC-3ML cells, wherein the upper panel shows the results of measurement using an anti-CD9 antibody and the lower panel shows the results of measurement using an anti-CD63 antibody, respectively. Dotted lines in the graphs show values of negative control (Allstar), and broken lines show values of positive control (RAB27A).
[FIG. 4] FIG. 4 is graphs showing the results of evaluation of inhibition of exosome secretion by siRNA of NAPG. Left zone is the results for PC-3ML cells, and right zone is the results for MDA-MB-231D3H2LN cells.
[FIG. 5] FIG. 5 is a graph of the results of evaluation of inhibition of exosome secretion by siRNA using MDA-MB-231D3H2LN cells. The inhibition of exosome secretion was measured by using CD9 antibody.
[FIG. 6] FIG. 6 is graphs showing the results of evaluation of actions inhibiting cancer metastasis in an *in vivo* model. (A) is a graph of measurements of change in tumor sizes of primary focus by administration of siRNA, and (B) is a graph showing the number of micro metastasis to the lungs.

### MODES FOR CARRYING OUT THE INVENTION

The exosome secretion inhibitor of the present invention is greatly characterized in that the exosome secretion inhibitor contains a substance suppressing expression of NAPG gene, HINT3 gene or GXYLT1 gene, or a substance suppressing activity of NAPG protein, HINT3 protein or GXYLT1 protein. Incidentally, terms used herein are used in the meaning ordinarily used in the field, unless otherwise noted particularly.

It is known that particular biomarkers are involved in various cancers. On the other hand, it is known that an increase in the amount of exosome secretion is involved in the progress or metastasis of cancer. In view of the above, when the present inventors have studied remarking on biomarkers capable of regulating exosome secretion, it has been found that the exosome secretion can be suppressed by suppressing expression of NAPG gene, HINT3 gene or GXYLT1 gene, which in turn makes it possible to suppress the progress or metastasis of cancer.

The "NAPG gene" as used herein means a gene encoding human NAPG protein. A nucleotide sequence of human NAPG gene and an amino acid sequence of human NAPG protein are known, and for example, each is registered in GenBank and published as GenBank Accession ID: CR536554 as a nucleotide sequence of NAPG gene (SEQ ID NO: 1) and GenBank Accession ID: CAG38791 as an amino acid sequence of human NAPG protein (SEQ ID NO: 2). Conventionally, there have been only findings that expression level of NAPG gene is increased in endothelial cells in ovarian cancer and that NAPG gene is involved in bipolar disorder or vesiculation; however, the present inventors have found for the first time an action of suppressing exosome secretion by NAPG gene.

The "HINT3 gene" as used herein means a gene encoding human HINT3 protein. A nucleotide sequence of human HINT3 gene and an amino acid sequence of human HINT3 protein are known, and for example, each is registered in GenBank and published as GenBank Accession ID: NM_138571 as a nucleotide sequence of HINT3 gene (SEQ ID NO: 3) and GenBank Accession ID: NP_612638 as an amino acid sequence of human HINT3 protein (SEQ ID NO: 4). While it is reported that HINT3 gene functions as a hydrolase of phosphoramidic acid, the function thereof is not nearly known, and the present inventors have firstly found a suppression action of exosome secretion by HINT3 gene.

The "GXYLT1 gene" as used herein means a gene encoding human GXYLT1 protein. A nucleotide sequence of human GXYLT1 gene and an amino acid sequence of human GXYLT1 protein are known, and for example, each is registered in GenBank and published as GenBank Accession ID: NM_173601 as a nucleotide sequence of GXYLT1 gene (SEQ ID NO: 5) and GenBank Accession ID: NP_775872 as an amino acid sequence of human GXYLT1 protein (SEQ ID NO: 6). While it is reported that GXYLT1 gene functions as a polysaccharide synthase, a detailed analysis has not been carried out, and the present inventors have found for the first time an action of suppressing exosome secretion by GXYLT1 gene.

The human NAPG protein as used herein includes not only a protein consisting of the amino acid sequence of SEQ ID NO: 2, but also a mutant which may be generated in a human individual, including a protein in which one or more amino acids are deleted, substituted and/or added in the protein consisting of the sequence of SEQ ID NO: 2, generated by modifications based on polymorphism or mutation. However, these mutant human NAPG proteins have functions equivalent to those of the protein consisting of the amino acid sequence of SEQ ID NO: 2.

The "NAPG gene" as used herein includes not only a gene consisting of the nucleotide sequence of SEQ ID NO: 1, but also a mutant which may be generated in a human individual, including, for example, a gene in which one or more bases are deleted, substituted and/or added in the gene consisting of the nucleotide sequence of SEQ ID NO: 1, generated by modifications based on polymorphism or mutation. Further, the "NAPG gene" includes a mutant consisting of a nucleotide sequence having an identity of 80% or more, for example, 85% or more, 90% or more, 95% or more, 97% or more, 98% or more, 99% or more, 99.5% or more, 99.7% or more, or 99.9% or more of the nucleotide sequence of SEQ ID NO: 1. The identity of a nucleotide sequence can be determined by utilizing a known algorism such as BLAST or FASTA. Further, the "NAPG gene" includes a mutant consisting of a nucleotide sequence hybridizing to the gene consisting of the nucleotide sequence of SEQ ID NO: 1 under stringent conditions. Here, in the present specification, examples of stringent hybridization conditions include, for example, usually conditions of "1 × SSC, 0.1% SDS at 37°C" or so as washing conditions after hybridization. It is preferred that a complementary chain maintains a hybridized state with a positive chain to be subject even if the complementary chain is washed under the above conditions. More stringent hybridization conditions include, without being particularly limited to, washing conditions of "0.5 × SSC, 0.1% SDS at 42°C" or so, and further more stringent hybridization conditions include washing conditions of "0.1 × SSC, 0.1% SDS at 65°C" or so. In addition, the hybridization can be carried out in accordance with the methods described in Molecular Cloning: A Laboratory Manual, Second Edition (1989) (Cold Spring Harbor Laboratory Press), Current Protocols in Molecular Biology (1994) (Wiley-Interscience), DNA Cloning 1: Core Techniques, A Practical Approach, Second Edition (1995) (Oxford University Press), and the like. However, these mutant genes encode proteins having functions equivalent to those of the protein consisting of the amino acid sequence of SEQ ID NO: 2.

The human HINT3 protein used herein includes not only a protein consisting of the amino acid sequence of SEQ ID NO: 4, but also a mutant which may be generated in a human individual, including a protein in which one or more amino acids are deleted, substituted and/or added in the protein consisting of the sequence of SEQ ID NO: 4, generated by modifications based on polymorphism or mutation. However, these mutant human HINT3 proteins have functions equivalent to those of the protein consisting of the amino acid sequence of SEQ ID NO: 4.

The HINT3 gene as used herein includes not only a gene consisting of the nucleotide sequence of SEQ ID NO: 3, but also a mutant which may be generated in a human individual, including, for example, a gene in which one or more bases are deleted, substituted and/or added in the gene consisting of the nucleotide sequence of SEQ ID NO: 3, generated by modifications based on polymorphism or mutation. Further, the "HINT3 gene" includes a mutant consisting of a nucleotide sequence having an identity of 80% or more, for example, 85% or more, 90% or more, 95% or more, 97% or more, 98% or more, 99% or more, 99.5% or more, 99.7% or more or 99.9% or more of the nucleotide sequence of SEQ ID NO: 3. The identity of the nucleotide sequence can be determined in the same manner mentioned above. Further, the "HINT3 gene" includes a mutant consisting of a nucleotide sequence hybridizing to the gene consisting of the nucleotide sequence of SEQ ID NO: 3 under stringent conditions. However, these mutant genes encode proteins having functions equivalent to those of the protein consisting of the amino acid sequence of SEQ ID NO: 4.

The human GXYLT 1 protein used herein includes not only a protein consisting of the amino acid sequence of SEQ ID NO: 6, but also a mutant which may be generated in a human individual, including proteins in which one or more amino acids are deleted, substituted and/or added in the protein consisting of the sequence of SEQ ID NO: 6, generated by modifications based on polymorphism or mutation. However, these mutant human GXYLT1 proteins have functions equivalent to those of the protein consisting of the amino acid sequence of SEQ ID NO: 6.

The "GXYLT1 gene" as used herein includes not only a gene consisting of the nucleotide sequence of SEQ ID NO: 5, but also a mutant which may be generated in a human individual, including, for example, a gene in which one or more bases are deleted, substituted and/or added in the gene consisting of the nucleotide sequence of SEQ ID NO: 5, generated by modifications based on polymorphism or mutation. Further, the "GXYLT 1 gene" includes a mutant consisting of a nucleotide sequence having an identity of 80% or more, for example, 85% or more, 90% or more, 95% or more, 97% or more, 98% or more, 99% or more, 99.5% or more, 99.7% or more or 99.9% or more of the nucleotide sequence of SEQ ID NO: 5. The identity of the nucleotide sequence can be determined in the same manner mentioned above. Further, the "GXYLT1 gene" includes a mutant consisting of a nucleotide sequence hybridizing to the gene consisting of the nucleotide sequence of SEQ ID NO: 5 under stringent conditions. However, these mutant genes encode proteins having functions equivalent to those of the protein consisting of the amino acid sequence of SEQ ID NO: 6.

The "substance suppressing expression of gene" as used herein is not particularly limited, so long as the substance is a substance suppressing transcription of mRNA of a target gene, a substance degrading mRNA transcribed, or a substance suppressing translation of a protein from mRNA. The substance includes a nucleic acid, and examples include siRNA, miRNA, an antisense oligonucleotide and a ribozyme, and an expression vector thereof. Among them, siRNA, miRNA, an antisense oligonucleotide, and an expression vector thereof are preferred, and siRNA and an antisense oligonucleotide are more preferred. The "substance suppressing expression of gene" includes a protein, a peptide, or other low-molecular compounds in addition to the above. Here, the target gene in the present invention is NAPG gene, HINT3 gene, or GXYLT1 gene.

The "siRNA" as used herein is an RNA molecule having a double stranded RNA moiety composed of from about 15 to 40 bases, in which the siRNA has a function of cleaving mRNA of a target gene having a sequence complementary to an antisense strand of the above siRNA to suppress expression of the target gene. In particular, the siRNA in the present invention is an RNA containing a double stranded RNA moiety consisting of a sense RNA strand consisting of a sequence homologous to continuous RNA sequence in mRNA of NAPG gene, HINT3 gene, or GXYLT1 gene, and an antisense RNA strand consisting of a sequence complementary to the sense RNA sequence. The design and production of the siRNA and mutant siRNA described later are within the scope of skill of one of ordinary skill in the art.

The "miRNA" as used herein refers to a RNA molecule having a double stranded RNA moiety composed of from about 21 to 25 bases, which has a function of suppressing expression of a target gene by binding to mRNA of a target gene. In particular, miRNA in the present invention is an RNA containing a double stranded RNA moiety composed of a sense RNA strand consisting of a sequence homologous to continuous RNA sequences in mRNA of NAPG gene, HINT3 gene, or GXYLT1 gene, and an antisense RNA strand consisting of a sequence complementary to the sense RNA sequence. The design and production of the miRNA and mutant siRNA set forth below are within the scope of skill of one of ordinary skill in the art.

The length of the double stranded RNA moiety of the siRNA is preferably from about 15 to 40 bases, more preferably from 15 to 30 bases, even more preferably from 15 to 25 bases, even more preferably from 18 to 23 bases, and still even more preferably from 19 to 21 bases as a base. In addition, the length of the double stranded RNA moiety of the miRNA is preferably from about 21 to 25 bases, and more preferably from 22 to 24 bases as a base. The end structures of a sense strand or an antisense strand of the siRNA and the miRNA are not particularly limited, and can be appropriately selected depending upon the purpose. For example, the end structure may be blunt ended or may have a protrusion end (overhang), and one having a 3' end protrusion is preferred. The siRNA and miRNA having an overhang composed of several bases, preferably from 1 to 3 bases, and more preferably 2 bases at a 3' end of the sense RNA strand and the antisense RNA strand are preferred because they are highly likely to have large effects of suppressing expression of a target gene. The kinds of bases of overhang are not particularly limited, and may be any one of bases constituting an RNA or bases constituting a DNA.

Further, the siRNA and the miRNA in which one or more nucleotides are deleted, substituted, inserted and/or added in one or both of the sense strand or the antisense strand of the above siRNA and miRNA can be also used in the exosome secretion inhibitor of the present invention. Here, the one or more bases are not particularly limited, and are preferably from 1 to 4 bases, more preferably from 1 to 3 bases, and even more preferably 1 or 2 bases. Concrete examples of the mutations include a mutation in which the number of bases of the overhang moiety at a 3' end is from 0 to 3; a mutation in which a nucleotide sequence of the overhang moiety at a 3' end is changed to other nucleotide sequences; a mutation in which the length of the above sense RNA strand is different from the length of the above antisense RNA strand by from 1 to 3 bases by having insertion, addition or deletion of bases; a mutation in which a base is substituted with another base in the sense strand and/or the antisense strand, and the like, but not limited thereto. However, it is required that the sense strand and the antisense strand can be hybridized in these mutants siRNA and miRNA, and that these mutants siRNA and miRNA have ability of suppressing gene expression equivalent to those of siRNA and miRNA without mutations.

Further, the siRNA and the miRNA may be a molecule in which either one end is closed, for example, siRNA and miRNA having a hairpin structure, a short hairpin RNA (shRNA). The shRNA is an RNA containing a sense strand RNA of a particular sequence of a target gene, an antisense strand RNA consisting of a sequence complementary to the sense strand RNA, and a linker sequence linking both the strands, in which the sense strand moiety and the antisense strand moiety are hybridized to form a double stranded RNA moiety.

It is desired that the siRNA and the miRNA do not exhibit so-called off-target effects during clinical use. The off-target effects refer to an action of suppressing expression of another gene partially having homology to the siRNA and the miRNA used other than the target gene. In order to avoid the off-target effects, it is possible to previously confirm that the candidates siRNA and miRNA do not have a cross-reaction by utilizing a DNA microarray or the like. In addition, the off-target effects can be avoided by confirming whether or not there are any genes containing parts having high homologies to the sequences of the candidates siRNA and miRNA other than the gene to be targeted, using known database provided by NCBI (National Center for Biotechnology Information) or the like.

In order to produce the siRNA and the miRNA of the present invention, known methods such as a method according to a chemical synthesis and a method using gene recombinant technology can be properly used. In the method according to synthesis, a double stranded RNA can be synthesized according to the ordinary method on the basis of the sequence information. In addition, in the method using a gene recombinant technology, an expression vector into which a sense strand sequence and an antisense strand sequence are incorporated is constructed, and the vector is introduced into a host cell, whereby each of a sense strand RNA and antisense strand RNA produced by transcription can then be generated by acquirement. In addition, a desired double stranded RNA can be produced by expressing shRNA forming a hairpin structure, which contains a sense strand RNA of a particular sequence of a target gene, an antisense strand RNA consisting of a sequence complementary to the sense strand RNA and a linker sequence linking both the strands.

The siRNA and the miRNA may have a DNA in a part of nucleic acids constituting the siRNA and miRNA, so long as the siRNA and the miRNA have activity of suppressing expression of a target gene. In addition, the siRNA and the miRNA may be a nucleic acid in which the entire or a part of nucleic acids constituting the siRNA and the miRNA is modified, so long as the siRNA and the miRNA have activity of suppressing expression of a target gene.

The modified nucleic acid means a nucleic acid having a structure different from a naturally occurring nucleic acid because of modification provided in nucleoside (base site or sugar site) and/or internucleoside linkage site. The "modified nucleoside" constituting the modified nucleic acids includes, for example, abasic nucleoside; arabinonucleoside, 2'-deoxyuridine, α-deoxyribonucleoside, β-L-deoxyribonucleoside, other nucleosides with sugar modification; peptide nucleic acid (PNA), phosphate group-bound peptide nucleic acid (PHONA), locked nucleic acid (LNA), morpholino nucleic acid, and the like. The above nucleoside with sugar modification includes a nucleoside with a sugar modification such as substituted pentose such as 2'-O-methylribose, 2'-deoxy-2'-fluororibose, 3'-O-methylribose; 1',2'-deoxyribose; arabinose; substituted arabinoses; and hexose and alpha-anomer. These nucleosides may be a modified base with modified at a basic site. The modified base includes, for example, pyrimidines such as 5-hydroxycytosine, 5-fluorouracil, and 4-thiouracil; purines such as 6-methyladenine and 6-thioguanosine; and other heterocyclic base, and the like.

The "modified internucleoside linkage" constituting the modified nucleic acid includes, for example, an alkyl linker, a glyceryl linker, an amino linker, a poly(ethylene glycol) linkage, a methyl phosphonate internucleoside linkage; a non-natural internucleoside linkage such as a methyl phosphonothioate, a phosphotriester, a phosphothiotriester, a phosphorothioate, a phosphorodithioate, a triester prodrug, a sulfonate, a sulfonamide, a sulfamate, formacetal, N-methyl hydroxylamine, a carbonate, a carbamate, a morpholino, a boranophosphonate, and a phosphoramidate.

An oligonucleotide complementary to the mRNA of a target gene is referred to as an "antisense oligonucleotide," and the antisense oligonucleotide forms a double strand with a gene to be targeted (mRNA), thereby suppressing an action of mRNA. The "antisense oligonucleotide" includes not only those completely complementary to a gene to be targeted (mRNA), and may have a few mismatches, so long as the antisense oligonucleotide can stably hybridize to mRNA.

The antisense oligonucleotide may be modified. The antisense oligonucleotide is less likely to be degraded in a living body by providing proper modification, whereby expression of a target gene can be more stably inhibited. The modified oligonucleotides described above include a modified antisense oligonucleotide such as an S-oligo form (phosphorothioate form), a C-5 thiazole form, a D-oligo form (phosphodiester form), an M-oligo form (methyl phosphonate form), a peptide nucleic acid form, a phosphodiester linkage form, a C-5 propynyl pyrimidine form, a 2-O-propylribose, and a 2'-methoxyethoxyribose form. Further, the antisense oligonucleotide may be substituted or modified with a sulfur atom in at least a part of oxygen atoms constituting a phosphate group. The antisense oligonucleotide described above is particularly excellent in nuclease resistance and affinity to RNA. The antisense oligonucleotide substituted or modified with a sulfur atom in at least a part of oxygen atoms constituting a phosphate group includes, for example, an oligonucleotide such as an S-oligo form.

The antisense oligonucleotide (or derivative thereof) can be synthesized by an ordinary method, and, for example, can be easily synthesized with a commercially available DNA synthesizer (for example, a synthesizer manufactured by Applied Biosystems, or the like). A synthesizing method includes a solid phase synthesizing method using a phosphoramidite, a solid phase synthesizing method using a hydrogen phosphonate, and the like.

While the "ribozyme" refers to an RNA having an enzymatic activity for cleaving a nucleic acid, recently it has been clarified that an oligo DNA having a nucleotide sequence of the enzymatic activity site also has activity of cleaving nucleic acids. Therefore, in the present specification, the ribozyme is used as a concept also encompassing a DNA, so long as a DNA has a sequence-specific nucleic acid cleavage activity. Concretely, the ribozyme is capable of specifically cleaving mRNA or initial transcripts encoding a target gene inside the coding region (including intron portions in a case of initial transcripts). Most useful ribozyme includes a self-splicing RNA found in an infective RNA such as viroid or virusoid, and a hammerhead form, a hairpin form or the like is known. The hammerhead form exhibits enzymatic activity in a size of about 40 bases or so, and only the target mRNA can be specifically cleaved by making several bases at both ends adjoining a portion taking a hammerhead structure (about 10 bases or so in total) into a sequence complementary to a desired cleavage site of mRNA. Further, when the ribozyme is used in the form of an expression vector containing a DNA encoding the ribozyme, in order to promote transition of the transcription product into a cytoplasm, the ribozyme can also be made into a hybrid ribozyme in which a sequence with a modified tRNA is further linked (Nucleic Acids Res., 29(13): 2780-2788 (2001)).

The substance suppressing expression of a target gene may be a nucleic acid molecule such as siRNA, miRNA, an antisense oligonucleotide or a ribozyme, and an expression vector encoding the nucleic acid molecule. In the expression vector, an oligonucleotide or a polynucleotide encoding the above nucleic acid molecule must be operably linked to a promoter capable of exhibiting a promoter activity in cells of a mammal to be administered. The promoter used is not particularly limited, so long as the promoter is capable of functioning in the mammal to be administered, and the promoter includes, for example, a pol III promoter (for example, a tRNA promoter, a U6 promoter, an H1 promoter), a promoter for mammal (for example, a CMV promoter, a CAG promoter, an SV40 promoter), and the like.

The expression vector preferably contains a transcription termination signal, i.e. a terminator region, downstream of an oligo(poly)nucleotide encoding the nucleic acid molecule. Further, the expression vector can additionally contain a selection marker gene for selection of transformed cells (a gene giving resistance against an agent such as tetracycline, ampicillin, kanamycin, hygromycin, or phosphinothricin, a gene complementing auxotrophic mutation, and the like).

A backbone vector used as the expression vector is not particularly limited, and includes, for example, a plasmid vector and a virus vector. A vector suitable for administration to a mammal such as a human includes a virus vectors such as retrovirus, adenovirus, adeno-associated virus, herpes virus, vaccinia virus, pox virus, polio virus, Sindbis virus, and Sendai virus.

The expression vector of the present invention includes an expression vector of siRNA, miRNA, an antisense oligonucleotide or a ribozyme, and among them, an expression vector of siRNA is preferred.

These "substances suppressing expression of genes" have some effects of inhibiting an exosome secretion, so that proliferation or metastasis of cancer cells in broad ranges including lung cancer, colorectal cancer, pancreatic cancer, breast cancer and the like is suppressed, or the cancer cells are diminished, whereby the substances can be used as a pharmaceutical composition.

The "substance suppressing activity of protein" as used herein is not particularly limited, so long as the substance is a substance suppressing activity of a target protein. Examples of the substance include a protein, a peptide, an antibody, other low-molecular compounds or the like. Among them, a peptide, an antibody and low-molecular compounds are preferred, and an antibody and low-molecular compounds are more preferred. Here, in the present invention, the target protein is NAPG protein, HINT3 protein or GXYLT 1 protein.

In the present invention, the substance suppressing expression of NAPG, HINT3 or GXYLT 1 gene or the substance suppressing activity of NAPG, HINT3 or GXYLT 1 protein can be used as an active ingredient of a pharmaceutical composition. The above pharmaceutical composition can be used as a pharmaceutical composition for treating cancer or a pharmaceutical composition for suppressing cancer metastasis by administering the composition to a living body.

Accordingly, the present invention also provides a pharmaceutical composition containing a substance suppressing expression of NAPG, HINT3 or GXYLT 1 gene or a substance suppressing activity of NAPG, HINT3 or GXYLT 1 protein as an active ingredient.

When the pharmaceutical composition of the present invention is used, for example, for cancer as a subject to be treated, the kinds of cancer are not particularly limited. In addition, the cancer may be solid cancer or invasive cancer. Concretely, the cancer includes, for example, urinary bladder cancer, breast cancer, colon cancer, colorectal cancer, renal cancer, liver cancer, lung cancer, small cell lung cancer, esophageal cancer, gallbladder cancer, ovarian cancer, pancreatic cancer, gastric cancer, cervical cancer, thyroid cancer, prostate cancer, squamous cancer, skin cancer, bone cancer, lymphoma, leukemia and brain tumor. Especially, the application of the pharmaceutical composition of the present invention is expected for breast cancer and colorectal cancer.

In addition, the pharmaceutical composition of the present invention can be used not only for treatment of cancer, but also for prevention of recurrence of cancer after treatment and suppression of metastasis. Accordingly, a preferred embodiment of the pharmaceutical composition of the present invention includes a pharmaceutical composition for treatment of cancer, a pharmaceutical composition for prevention of recurrence of cancer, and a pharmaceutical composition for suppression of cancer metastasis.

As the pharmaceutical composition of the present invention, both dosage forms of an oral administration and a parenteral administration can be adopted. In a case of a parenteral administration, the pharmaceutical composition can also be directly administered to a tumor site.

The pharmaceutical composition of the present invention can be produced into a formulation in accordance with a conventional method, and the pharmaceutical composition may contain a pharmaceutically acceptable carrier and an additive. The carrier and the additives as mentioned above include water, a pharmaceutically acceptable organic solvent, a collagen, a polyvinyl alcohol, a polyvinyl pyrrolidone, a carboxyvinyl polymer, sodium carboxymethyl cellulose, a sodium polyacrylate, sodium alginate, a water-soluble dextran, sodium carboxymethyl starch, pectin, methyl cellulose, ethyl cellulose, xanthan gum, gum arabic, casein, agar, a polyethylene glycol, diglycerol, glycerol, propylene glycol, Vaseline, a paraffin, stearyl alcohol, stearic acid, human serum albumin, mannitol, sorbitol, lactose, a surfactant accepted as a pharmaceutical additive, and the like.

The additives are selected alone or in a proper combination of two or more kinds among the above list depending upon the dosage forms of the pharmaceutical composition of the present invention. The dosage form can be administered as a tablet, a capsule, a fine powder, a powder, a granule, a solution, a syrup, or in a suitable dosage form in a case of an oral administration. In a case of parenteral administration, the dosage form includes a transpulmonary dosage form (for example, a form delivered by using a nebulizer or the like), a transnasal administration dosage form, a transcutaneous administration dosage form (for example, an ointment or a cream), an injectable dosage form and the like. In a case of an injectable dosage form, the dosage form can be systemically or locally administered by an intravenous injection, an intramuscular injection, an intraperitoneal injection, a subcutaneous injection or the like.

In a case where the substance suppressing expression of a target gene is a nucleic acid molecule such as siRNA, miRNA, an antisense oligonucleotide or a ribozyme, or an expression vector encoding the nucleic acid, it is possible to introduce the substance suppressing expression into a phospholipid vesicle such as a liposome to make the vesicle into a pharmaceutical composition of the present invention.

The dose of the pharmaceutical composition of the present invention varies depending upon age, sex, symptom, administration route, the number of administration or dosage form. A method of administration is properly selected depending upon age or symptom of a patient. An effective dose is preferably 0.01 µg to 1,000 mg, and more preferably 0.1 µg to 100 µg per administration per 1 kg of body weight. However, the above therapeutic agent is not limited to these doses.

In a further embodiment, the present invention provides:
(i) a method of treating cancer including administering a substance suppressing expression of NAPG, HINT3 or GXYLT1 gene, or a substance suppressing activity of NAPG, HINT3 or GXYLT1 protein;
(ii) a substance suppressing expression of NAPG, HINT3 or GXYLT1 gene, or a substance suppressing activity of NAPG, HINT3 or GXYLT1 protein which is usable in treatment of cancer; and
(iii) use of a substance suppressing expression of NAPG, HINT3 or GXYLT1 gene, or a substance suppressing activity of NAPG, HINT3 or GXYLT1 protein in the preparation of a therapeutic agent of cancer.

The above "substance suppressing expression of gene" or "substance suppressing activity of protein" is as explained above. In addition, in a case where siRNA is used as "a substance suppressing expression of gene," a preferred siRNA is as mentioned above.

In another embodiment, the present invention provide a method of screening an exosome secretion inhibitor, including using an exosome amount as an index to have a substance suppressing secretion of exosome as a candidate compound.

A substance to be tested subjected to the method of screening may be any known compounds and novel compounds, and the substance includes, for example, a nucleic acid, a saccharide, a lipid, a protein, a peptide, an organic low-molecular compound, a compound library produced using a combinatorial chemistry technique, a random peptide library produced in accordance with a solid phase synthesis or a phage display method, a naturally occurring component derived from microbes, animals, plants, marine organisms etc., and the like.

The method of screening the present invention preferably includes methods of the following embodiment 1 and embodiment 2. The screening method of the embodiment 1 includes the following steps (a1) to (c1):
(a1) contacting a cell of which exosome amount is measurable with a substance to be tested;
(b1) measuring the exosome amount in the cell contacted with the substance to be tested to compare the measurement values with an exosome amount in a control cell without contacting the substance to be tested; and
(c1) selecting the substance to be tested as a substance for inhibiting exosome secretion in a case where the exosome amount in the cell contacted with the substance to be tested is lowered than the exosome amount in the control cell without contacting the substance to be tested.

The method of the embodiment 2 includes the following steps (a2) to (c2):
(a2) contacting a cell of which exosome amount is measurable with a substance to be tested;
(b2) measuring the exosome amount in the cell contacted with the substance to be tested to compare the measurement values with the exosome amount previously measured before contacting the cell with the substance to be tested; and
(c2) selecting the substance to be tested as a substance for inhibiting exosome secretion in a case where the exosome amount in the cell contacted with the substance to be tested is lowered more than the exosome amount in the cell before contacting with the substance to be tested.

In the steps (a1) and (a2), the cell of which exosome amount is measurable and the substance to be tested are placed under contact conditions. The contact of the cell with the substance to be tested is carried out in a culture medium.

The cell of which exosome amount is measurable includes nearly all animal cells, and the examples thereof include a disease cell such as a cancer cell, and a normal cell such as a vascular endothelial cell, an epidermal cell, a nerve cell, and an immunological cell. Here, as these cells, an animal cell, for example, a mammalian cell of mouse, rat, hamster, guinea pig, rabbit, dog, monkey or human can be used, among which a cell derived from human is desired.

The cell culture medium in which a cell of which exosome amount is measurable is contacted with a substance to be tested is properly selected depending upon the kinds of cells used and the like, and the medium is, for example, minimum essential medium (MEM) containing from about 5 to about 20% of fetal bovine serum, Dulbecco's modified minimum essential medium (DMEM), RPMI1640 medium, 199 medium or the like. The culture conditions are also properly determined depending upon the kinds of cells usable and the like, and a pH of the medium is from about 6 to about 8, a culture temperature is usually from about 30° to about 40°C, and a culture time is from about 12 to about 144 hours.

In the step (b1), the exosome amounts of the cell contacted with the substance to be tested and the control cell without contacting the substance to be tested are measured, and in the step (b2), the exosome amounts of the cell contacted with the substance to be tested before and after the contact are measured. The measurement of exosome amounts can be carried out in accordance with a known method, and the amount can be calculated as an exosome amount per cell. Concretely, preferred examples include, for example, a mass spectrometry and a method using an antibody which is capable of specifically recognizing a marker to be measured. Here, the exosome amount in the control cell without contacting can be newly measured every time the exosome amount in the cell contacted with substance to be tested is measured, but a previously measured exosome amount can be also utilized. The measurement of the cell count can be carried out in accordance with a known method.

The mass spectrometry includes mass spectrometry using MALDI (matrix-assisted laser desorption ionization) type mass spectrometer, ESI (electrospray ionization) type mass spectrometer and the like, and the method may be a method of quantification from a partial peptide of a protein. Among them, MALDI-TOF MS using MALDI type spectrometer is preferred.

The method using an antibody, which is an immunoassay, includes, for example, Western blotting, radioimmunoassay (RIA), enzyme-linked immunosorbent assay (ELISA or EIA), luminescent immunoassay, fluorescent immunoassay (exoscreen or the like). As the antibodies used in the method, since antigens which are specifically expressed on a membrane surface of exosome (for example, CD9, CD63, CD147, EPCAM or the like) are known, antibodies capable of specifically binding to these antigens can be used (for example, WO 2013/099925). The antibody as described above can be produced in accordance with a method which is well known to one of ordinary skill in the art, and for example, a monoclonal antibody or a fragment thereof can be suitably used. In addition, the above monoclonal antibody or the fragment thereof can be labeled or formed into a solid phase in accordance with an ordinarily known method and used. Here, "a fragment of monoclonal antibody" as used herein means a part of the above-mentioned monoclonal antibody, the fragment having specific bindability against a target protein in the same manner as the above monoclonal antibody. Concrete examples include a Fab, a F(ab')₂, a Fab', a single chained antibody (scFv), a disulfide-stabilized antibody (dsFv), a dimerized V region fragment (Diabody), a peptide containing a CDR, and the like.

In the step (c1), the exosome amount in the cell contacted with the substance to be tested is compared to the exosome amount in the control cell without contacting the substance to be tested, and in the step (c2), the exosome amounts before and after contacting with the substance to be tested are compared. The comparison of the exosome amounts is preferably carried out based on the presence or absence of significant differences. For example, in a case where the exosome amount in the cell contacted with the substance to be tested is lowered than the exosome amount in the control cell without contacting the substance to be tested, or the exosome amount in the cell after contacting the substance to be tested is lowered than the exosome amount in the cell before contacting with the substance to be tested in a statistically significant amount, preferably by two times or more, more preferably three times or more, even more preferably four times or more, and even more preferably five times or more, it is preferable to select the substance to be tested as a substance for inhibiting exosome secretion. As a test method in statistical processing, a known test method in which the presence or absence of significance can be judged may be properly used, and are not particularly limited thereto. For example, Student t test and multiple comparison test can be used.

In addition, in another embodiment, the present invention provides a method of screening a therapeutic agent for cancer, including using an exosome amount as an index to have a substance suppressing secretion of exosome as a candidate compound. The concretely used sample and method are same as in the method of screening an exosome secreting inhibitor of the present invention.

### EXAMPLES

The present invention will be described based on Examples, which are exemplifications for more fully understanding the present invention, without intending to limit the scope of the present invention to these Examples. Here, "ordinary temperature" used in the present Examples means from 15° to 30°C.

### Example 1 (Search of miRNA Having Inhibitory Action of Exosome Secretion)

The amount 1 × 10⁴ cells of PC-3ML cells (Xenogen) or MDA-MB-231D3H2LN cells (Xenogen) were seeded to each well in a serum-containing medium in a 96-well plate. Next day, the culture supernatant was exchanged with advanced RPMI (aRPMI; serum free, antibiotics free) (90 µL/well).

To each well of a separately furnished 96-well plate (PCR plate), 5 µL of 2 µM miRNA and 10 µL of aRPMI were mixed, for each miRNA in a micro RNA library (Accu Target Human Library mimic).

Next, DharmaFECT1 Transfection Reagent (Thermo Scientific) was diluted with aRPMI so as to have a concentration of 10 µL/well, and the solution was incubated at an ordinary temperature for 5 minutes, and aRPMI was added thereto so as to have a concentration of 90 µL/well. The solution was added in an amount of 90 µL per well of a PCR plate to which the above miRNA was injected, and the mixture was incubated at an ordinary temperature for 30 minutes. After the incubation at an ordinary temperature for 30 minutes, 90 µL of the incubated mixture was added to each well of a 96-well plate in which PC-3ML cells or MDA-MB-231D3H2LN cells were seeded. Next day, the culture supernatant was removed, and new aRPMI was added thereto in an amount of 60 µL. After additional 24 hours, 10 µL of the culture supernatant was used for quantification of the amount of exosome secretion, and the remaining supernatant was used for quantification of the cell count. Here, each quantification was carried out in accordance with the following methods.

### <Quantification of Exosome Amount>

The collected culture supernatant is added to each well of a 96-well white plate in an amount of 10 µL each.

Biotinylated anti-CD9 antibodies (SHIONOGI & Co., Ltd., Clone 12A12) and biotinylated anti-CD63 antibodies (SHIONOGI & Co., Ltd., Clone 8A12) are diluted with AlphaLISA Universal Buffer (PerkinElmer) to a concentration of 5 nM. On the other hand, anti-CD9 antibodies (SHIONOGI & Co., Ltd., Clone 12A12) and anti-CD63 antibodies (SHIONOGI & Co., Ltd., Clone 8A12), each is conjugated with AlphaLISA Acceptor Beads, are diluted with AlphaLISA Universal Buffer (PerkinElmer) to a concentration of 50 µg/mL.

To the culture supernatant are added 10 µL of each of 5 nM biotinylated antibodies and 50 µg/mL antibodies bound to AlphaLISA Acceptor Beads, and the mixture is incubated at 37°C for 1 hour under shading conditions.

The amount 5 mg/mL AlphaScreen streptavidin donor beads (PerkinElmer) is diluted 62.5 times with AlphaLISA Universal Buffer, the diluted solution is added to each well in an amount of 25 µL, TopSeal-A is adhered to the plate and incubated at 37°C for 30 minutes under shading conditions, and the fluorescent intensity is measured with Enspire.

### < Quantification of Cell Count >

To the collected culture supernatant is added an MTS solution (Dojindo, Cell Counting Kit-8) so as to have a concentration of 50 µL/well, and the mixture is incubated at 37°C for 1 hour to measure an increase in cell count.

The exosome amount obtained was divided by the cell count to calculate an exosome secretion amount per cell count, to compare the effects of 2,042 kinds of miRNAs in a micro RNA library.

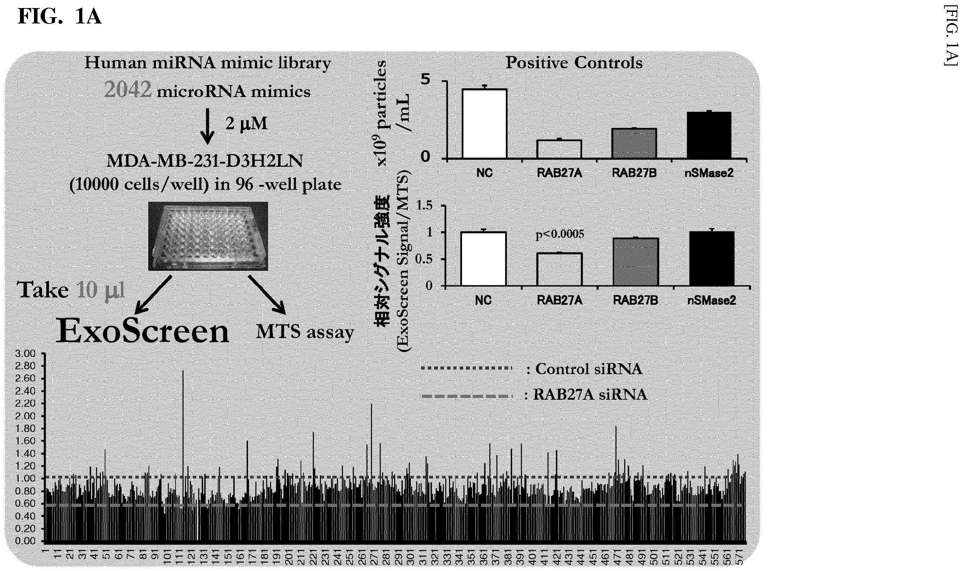

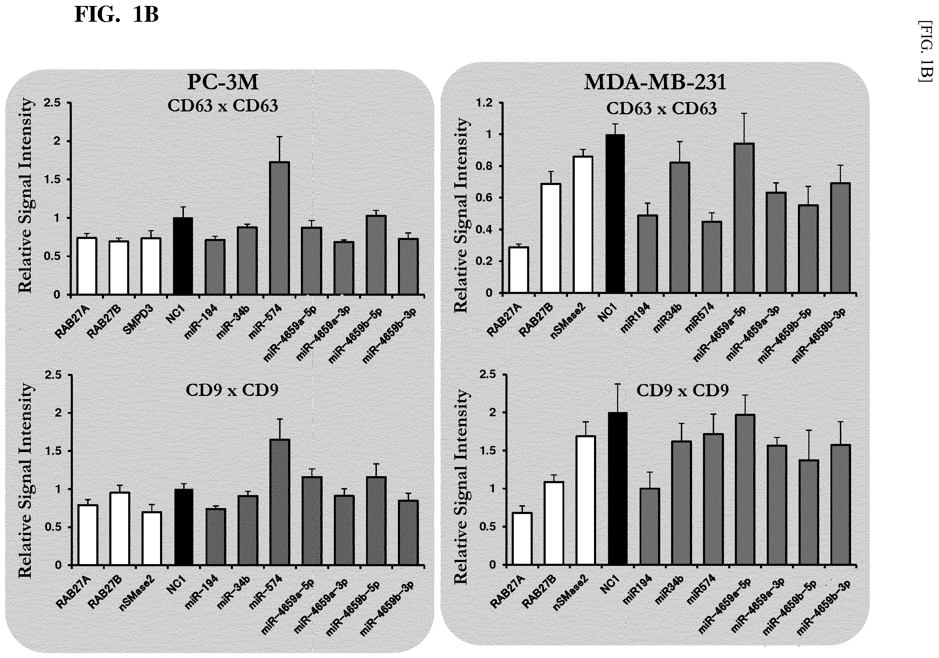

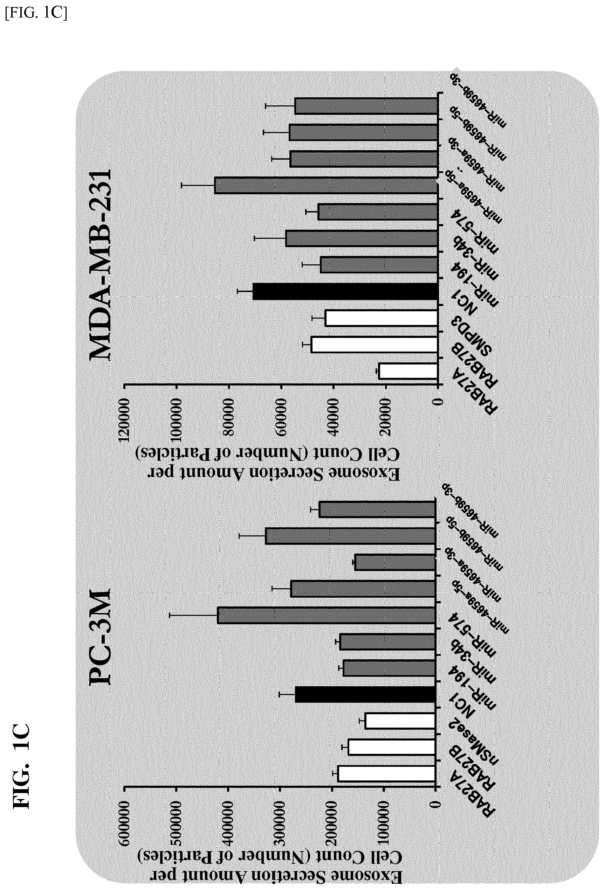

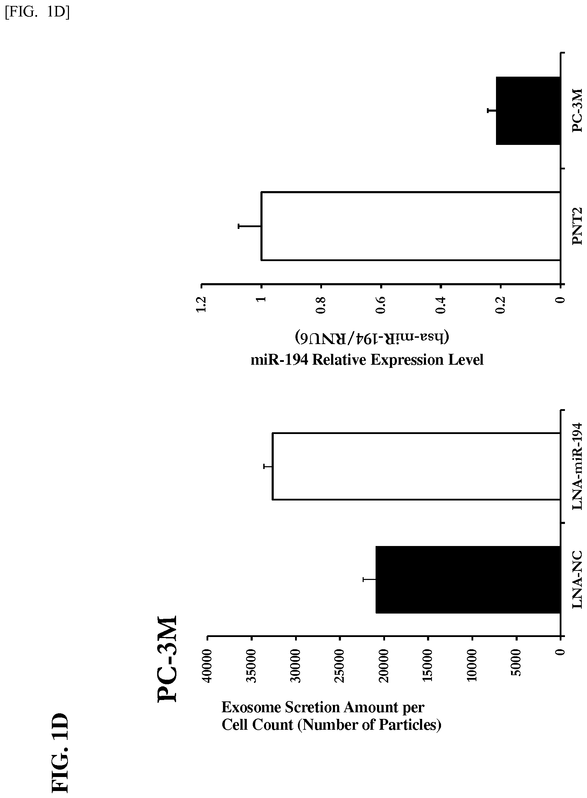

As a result of the comparison, it was found that miR-194 has suppressing action of exosome secretion (FIGs. 1A to D).

### Example 2 (Identification of Target Gene of miR-194)

The amount 1 × 10⁴ cells of PC-3ML cells or MDA-MB-231D3H2LN cells were seeded to each well in a serum-containing medium in a 6-well plate. Next day, the culture supernatant was exchanged with advanced RPMI (aRPMI; serum free, antibiotics free) (2 mL/well).

To a 1.5 mL tube were added 100 µL each of 2 µM miR-194mimic (Ambion) or miR-194 LNA, and 100 µL of aRPMI. DharmaFECT1 Transfection Reagent was furnished to have a concentration of 6 µL/well, the solution was diluted with aRPMI so as to have a concentration of 10 µL/well, the diluted solution was incubated at an ordinary temperature for 5 minutes. After termination of incubation, 400 µL of the solution was added to cells in each well.

Next day, the culture supernatant was removed, and new aRPMI was added thereto in an amount of 4 mL. After additional 24 hours, the culture supernatant was collected to calculate an exosome secretion amount per cell count in the same manner as in Example 1. In addition, the transfected cells were collected and RNA extraction was carried out using miRNeasy mini kit (QIAGEN), to compare expression of each of genes by a microarray with a tip manufactured by Agilent.

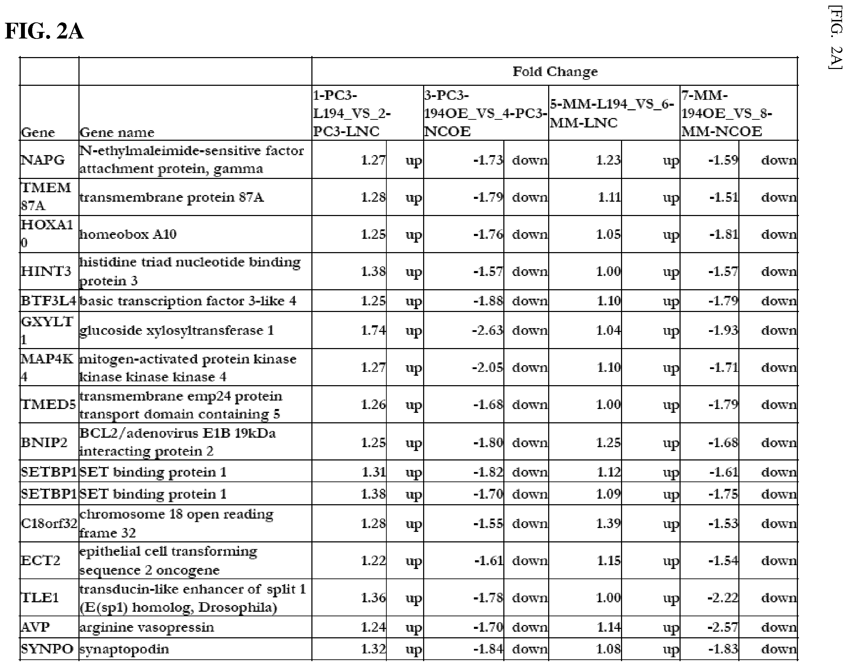

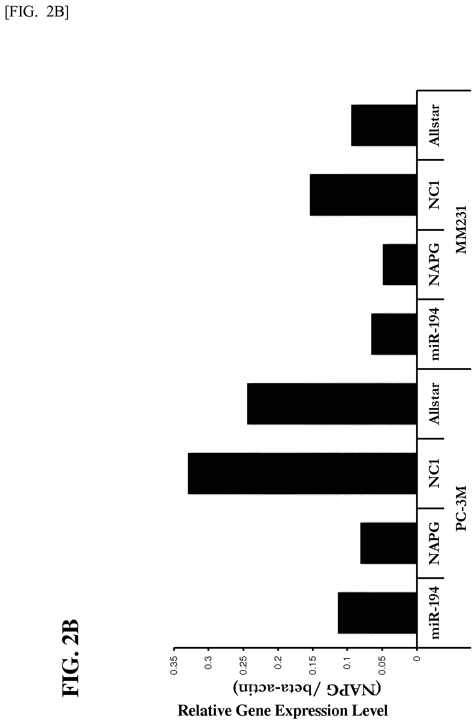

Since the gene of which expression level is lowered by administering miR-194 mimic and expression level is increased by administering miR-194 LNA is a candidate for the target gene of miR-194, genes such as NAPG were found as candidates for target genes of mir-194 (FIGs. 2A and B).

### Example 3 (Suppression of Exosome Secretion by siRNA of NAPG and TMED5)

The amount 1 × 10⁴ cells of PC-3ML cells or MDA-MB-231D3H2LN cells were seeded to each well in a serum-containing medium in a 96-well plate. Next day, the culture supernatant was exchanged with advanced RPMI (90 µL/well).

Each of 10 µL of siRNA (2 µM) against NAPG and TMED which were listed as candidates for target genes (see, FIG. 2A) and 10 µL of aRPMI were mixed to a separately furnished 96-well plate.

Next, DharmaFECT1 Transfection Reagent was diluted with aRPMI so as to have a concentration of 10 µL/well, the diluted solution was incubated at an ordinary temperature for 5 minutes, and aRPMI was added thereto so as to have a concentration of 90 µL/well. The solution was added thereto in an amount of 90 µL per well of a PCR plate, the mixture was incubated at an ordinary temperature for 30 minutes. After incubation at an ordinary temperature for 30 minutes, 90 µL of solution was added to each well of a 96-well plate in which PC-3ML cells or MDA-MB-231D3H2LN cells were seeded. Next day, the culture supernatant was removed and new aRPMI was added thereto in an amount of 60 µL. After additional 24 hours, 10 µL of the culture supernatant was used for quantification of an exosome secretion amount, and the remaining culture supernatant was used for quantification of the cell count. Each quantification was carried out in the same manner as in Example 1.

The exosome amount obtained was divided by the cell count to calculate an exosome secretion amount per cell count, to compare the effects of miRNAs against each target gene which was listed as a candidate.

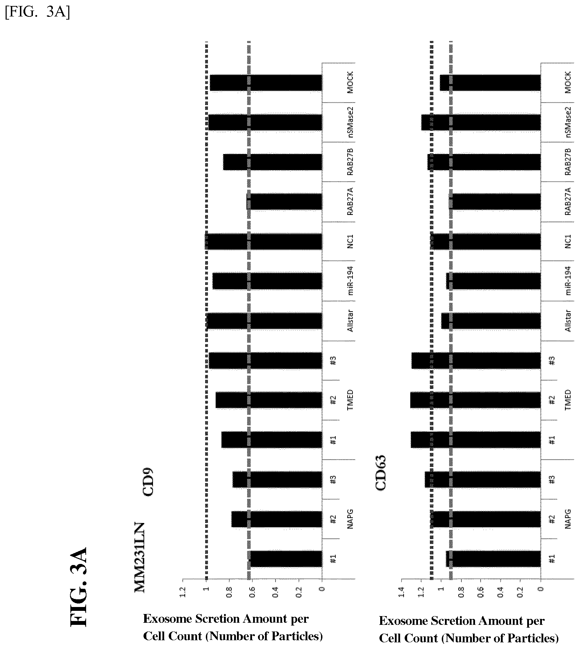

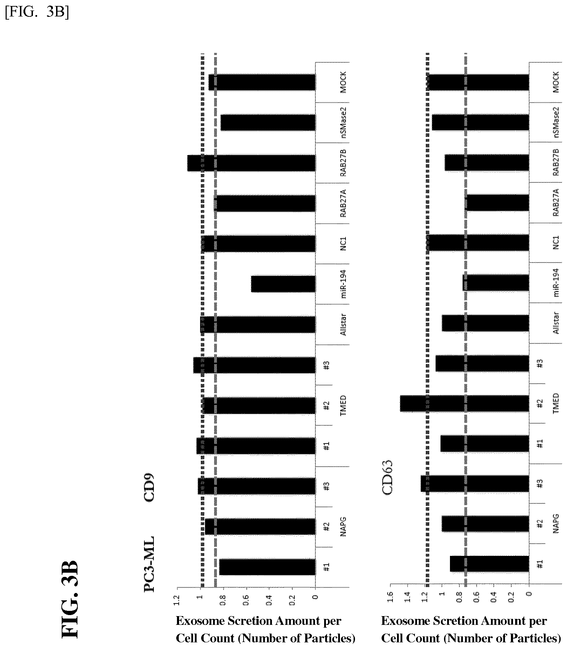

As a result of the comparison, it was found that the siRNA for NAPG has remarkable suppressing action of exosome secretion amount (FIGs. 3A and B).

### Example 4 (Measurement of Involvement of siRNA for NAPG in Exosome Secretion)

The amount 5 × 10⁵ cells of PC-3ML cells or MDA-MB-231D3H2LN cells were seeded to each well in a serum-containing medium in a 6-well plate. Next day, the culture supernatant was exchanged with advanced RPMI (2 mL/well).

To a 1.5 mL tube were added 100 µL of siRNA for 2 µM NAPG and 100 µL of aRPMI.

Next, DharmaFECT1 Transfection Reagent was diluted with aRPMI so as to have a concentration of 200 µL/well, the diluted solution was incubated at an ordinary temperature for 5 minutes, and the incubated solution was added to the tube containing each siRNA. After incubation at an ordinary temperature for 30 minutes, 400 µL of the mixture was added to each cell in wells.

Next day, the culture supernatant was removed, and new aRPMI was added thereto in an amount of 4 mL. After additional 24 hours, an entire amount of the culture supernatant was collected. The remaining cells were collected and the cell count was measured and counted with a hemacytometer. In addition, the collected culture supernatant was centrifuged at 2,000 × g for 10 minutes, the supernatant was collected, and the collected culture supernatant was filtrated with a 0.22 µm filter. The filtrated culture supernatant was ultracentrifuged at 100,000 × g for 70 min at 4°C. The supernatant was discarded, and the centrifuged product was washed with PBS at 100,000 × g for 70 min. The supernatant was discarded, 200 µL of PBS was added to a ultracentrifuge tube, exosome was collected, and an exosome amount was quantified with Nanosight or microBCA assay (Pierce).

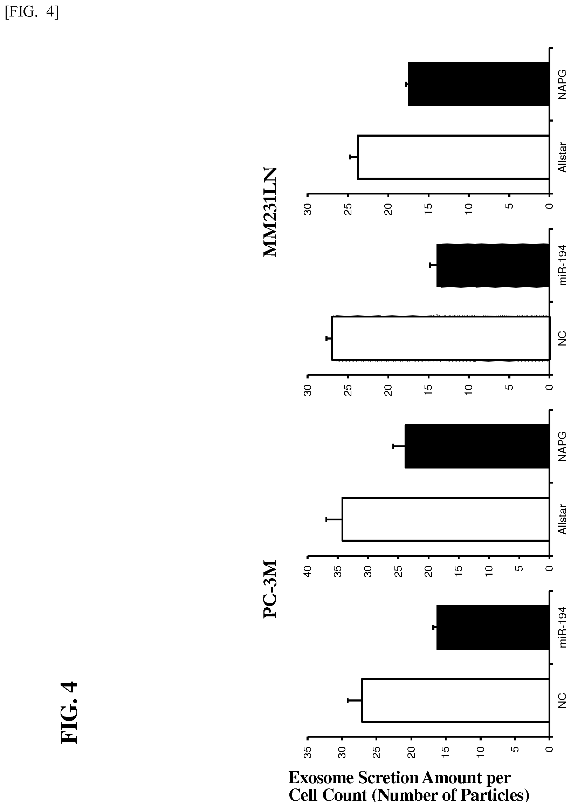

As a result, it was found that siRNA for NAPG suppresses the secretion of exosome (FIG. 4).

### Example 5 (Measurement of Involvement of siRNAs for HINT3 and GXYLT1 in Exosome Secretion)

The same analysis as in Example 3 was carried out for HINT3 and GXYLT1 genes which were found as candidates for the target genes of mir-194 in Example 2.

Concretely, the amount 1 × 10⁴ cells of MDA-MB-231D3H2LN cells were seeded to each well in a serum-containing medium in a 96-well plate. Next day, the culture supernatant was exchanged with advanced RPMI (90 µL/well).

To a separately furnished 96-well plate, 10 µL each of siRNAs against 2 µM HINT3 or GXYLT1 and 10 µL of aRPMI were mixed.

Next, DharmaFECT1 Transfection Reagent was diluted with aRPMI so as to have a concentration of 10 µL/well, the diluted solution was incubated at an ordinary temperature for 5 minutes, and aRPMI was added thereto so as to have a concentration of 90 µL/well. The solution was added to wells in an amount of 90 µL per well of a PCR plate, and the well was incubated at an ordinary temperature for 30 minutes. After the incubation at an ordinary temperature for 30 minutes, 90 µL each of an incubated mixture was added to each well of a 96-well plate in which MDA-MB-231D3H2LN cells were seeded. Next day, the culture supernatant was removed, and new aRPMI was added thereto in an amount of 60 µL. After additional 24 hours, 10 µL of the culture supernatant was used for quantification of an exosome secretion amount, and the remaining culture supernatant was used for quantification of the cell count. Each quantification was carried out in the same manner as in Example 1.

The obtained exosome amount was divided by the cell count to calculate an exosome secretion amount per the cell count, to compare the effects of siRNAs against each target gene which was listed as a candidate.

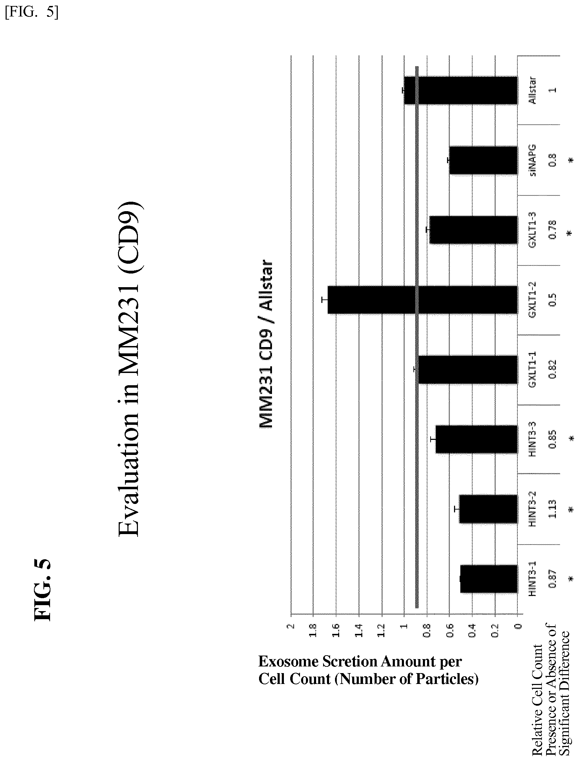

As a result of the comparison, it was found that siRNA for HINT3 and GXYLT1 have remarkable suppression action of exosome secretion (FIG. 5).

### Example 6 (Suppression of Cancer Metastasis in in vivo Model by siRNA for NAPG)

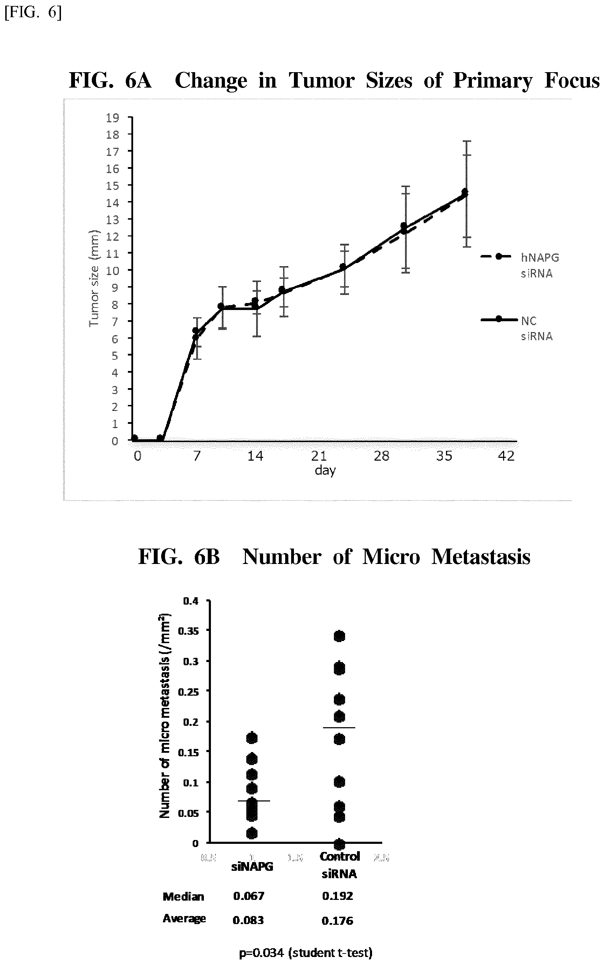

MDA-MB-231D3H2LN cells were transplanted into a mammary gland of SCID mice in an amount of 1 × 10⁶ cells/mouse (10 mice per group). siRNA against NAPG and siRNA which was a negative control (AllStars, QUIAGEN) were administered into a tumor with an *in vivo-*jetPEI (Polyplus-transfection) in an amount of 20 µg/mouse, twice per week after one week of the transplantation (for 3 weeks, 6 times administration in total). A tumor size in primary lesion was measured with a caliper once per week (FIG. 6A). After 44 days from the transplantation, the mice were anatomized, and an evaluation regarding the extent of metastasis to the lungs was made. A section of a lung tissue was prepared, and subjected to immunohistochemistry using an anti-human vimentin antibody. The number of micro metastasis which was detected as positive with the same antibody in each mouse was measured, to calculate the number of micro metastasis per 1 mm² (FIG. 6B). As a result, in mice administered with siRNA against NAPG, a difference of a tumor size of primary focus was not found, but the number of micro metastasis to a lung was significantly reduced, as compared to the mice administered with the negative control siRNA.

### INDUSTRIAL APPLICABILITY

The present invention is applicable in the field of a medicament, particularly in the fields of development and manufacture of a carcinostatic agent.

### SEQUENCE LISTING FREE TEXT

SEQ ID NO: 1 of Sequence Listing is a nucleotide sequence of mRNA of human NAPG.
SEQ ID NO: 2 of Sequence Listing is a polypeptide of human NAPG. SEQ ID NO: 3 of Sequence Listing is a nucleotide sequence of mRNA of human HINT3.
SEQ ID NO: 4 of Sequence Listing is a polypeptide of human HINT3.
SEQ ID NO: 5 of Sequence Listing is a nucleotide sequence of mRNA of human GXLT1.
SEQ ID NO: 6 of Sequence Listing is a polypeptide of human GXLT1.

## Claims

1. An exosome secretion inhibitor comprising a substance suppressing expression of NAPG, HINT3 or GXYLT1 gene, or a substance suppressing activity of NAPG, HINT3 or GXYLT1 protein.

2. The exosome secretion inhibitor according to claim 1, wherein at least one of the substance suppressing expression of NAPG, HINT3 or GXYLT1 gene, or the substance suppressing activity of NAPG, HINT3 or GXYLT1 protein is a nucleic acid.

3. The exosome secretion inhibitor according to claim 1 or 2, wherein the substance suppressing expression of the gene is one or more members selected from the group consisting of siRNA, miRNA, an antisense oligonucleotide and a ribozyme of the gene and an expression vector thereof.

4. A pharmaceutical composition comprising a substance suppressing expression of NAPG, HINT3 or GXYLT1 gene, or a substance suppressing activity of NAPG, HINT3 or GXYLT1 protein.

5. The pharmaceutical composition according to claim 4, wherein at least one of the substance suppressing expression of NAPG, HINT3 or GXYLT1 gene, or the substance suppressing activity of NAPG, HINT3 or GXYLT1 protein is a nucleic acid.

6. The pharmaceutical composition according to claim 4 or 5, wherein the substance suppressing expression of gene is one or more members selected from the group consisting of siRNA, miRNA, an antisense oligonucleotide and a ribozyme of the gene and an expression vector thereof.

7. The pharmaceutical composition according to any one of claims 4 to 6, for use in the treatment of cancer or the suppression of cancer metastasis.

8. The pharmaceutical composition according to claim 7, wherein the cancer is colorectal cancer or breast cancer.

9. An exosome secretion inhibitor comprising miR-194.

10. A method of screening a substance for inhibiting exosome secretion, comprising using expression of NAPG, HINT3 or GXYLT1 gene or activity of NAPG, HINT3 or GXYLT1 protein as an index to make a substance suppressing the expression or suppressing the activity a candidate compound.

11. The method of screening according to claim 9, comprising the following steps (a1) to (c1):
(a1) contacting a cell of which exosome amount is measurable with a substance to be tested;
(b1) measuring the exosome amount in the cell contacted with the substance to be tested to compare the measurement values with the exosome amount in a control cell without contacting with the substance to be tested; and
(c1) selecting the substance to be tested as a substance for inhibiting exosome secretion in a case where the exosome amount in the cell contacted with the substance to be tested is lowered than the exosome
amount in a control cell without contacting the substance to be tested.

12. The method of screening according to claim 10, comprising the following steps (a2) to (c2):
(a2) contacting a cell of which exosome amount is measurable with a substance to be tested;
(b2) measuring the exosome amount in the cell contacted with the substance to be tested to compare the measurement values with the exosome amount in the cell previously measured before contacting with the substance to be tested; and
(c2) selecting the substance to be tested as a substance for inhibiting an exosome secretion in a case where the exosome amount in the cell contacted with the substance to be tested is lowered than the exosome amount in the cell before contacting with the substance to be tested.

13. A method of treating cancer, comprising administering a therapeutically effective amount of an exosome secretion inhibitor as defined in any one of claims 1 to 3 or a pharmaceutical composition as defined in any one of claims 4 to 8 to an individual in need thereof.

14. A method of suppressing cancer metastasis, comprising administering a therapeutically effective amount of an exosome secretion inhibitor as defined in any one of claims 1 to 3 or a pharmaceutical composition as defined in any one of claims 4 to 8 to an individual in need thereof.
